# EUROPÄISCHE PATENTSCHRIFT

(11) **EP 1 537 246 B1**
(45) Veröffentlichungstag und Bekanntmachung des Hinweises auf die Patenterteilung: **11.11.2009**
(21) Anmeldenummer: 03797298.1
(22) Anmeldetag: 11.09.2003
(51) Int. Cl.: C12Q 1/68

(54) **Verfahren zur Amplifikation genetischer Information mittels an mehrere Stellen im Genom bindende Primer**
Method for the amplification of genetic information employing primers binding at multiple sites in the genome
Procédé d'amplification d'informations génétiques utilisant des oligonucléotides se liant aux sites multiples dans le génome

(30) Priorität: 12.09.2002 DE 10242359
(43) Veröffentlichungstag der Anmeldung: 08.06.2005
(73) Patentinhaber: Olympus Life Science Research Europa GmbH, 81377 München (DE)
(72) Erfinder: MANN, Wolfgang, 95512 Neudrossenfeld (DE)
(74) Vertreter: Manitz, Finsterwald & Partner GbR
(86) Internationale Anmeldenummer: PCT/EP2003/010132
(87) Internationale Veröffentlichungsnummer: WO 2004/027089

(56) Entgegenhaltungen:
- WO-A-00/24925
- WO-A-02/29066
- WO-A-92/13101
- US-A- 5 817 462
- MCCARTHY LINDA ET AL: "Efficient high-resolution genetic mapping of mouse interspersed repetitive sequence PCR products, toward integrated genetic and physical mapping of the mouse genome" PROCEEDINGS OF THE NATIONAL ACADEMY OF SCIENCES OF THE UNITED STATES, Bd. 92, Nr. 12, 1995, Seiten 5302-5306, XP002265941 1995 ISSN: 0027-8424
- HIMMELBAUER H ET AL: "Interspersed repetitive sequence (IRS)-PCR for typing of whole genome radiation hybrid panels." NUCLEIC ACIDS RESEARCH. ENGLAND 15 JAN 2000, Bd. 28, Nr. 2, 15. Januar 2000 (2000-01-15), Seite e7 XP002265942 ISSN: 1362-4962
- LEDBETTER S A ET AL: "RAPID ISOLATION OF DNA PROBES WITHIN SPECIFIC CHROMOSOME REGIONS BYINTERSPERSED REPETITIVE SEQUENCE POLYMERASE CHAIN REACTION" GENOMICS, ACADEMIC PRESS, SAN DIEGO, US, Bd. 6, 1990, Seiten 475-481, XP000283124 ISSN: 0888-7543
- ARMSTRONG B. ET AL: 'SUSPENSON ARRAYS FOR HIGH THROUGHPUT, MULTIPLEXED SINGLE NUCLEOTIDE POLYMORPHISM GENOTYPING' CYTOMETRY Bd. 40, Nr. 2, 01 Juni 2000, ALAN LISS, NEW YORK, US, Seiten 102 - 108, XP001106772

## Beschreibung

Die vorliegende Erfindung betrifft Verfahren zur Bestimmung der relativen Häufigkeit abgrenzbarer Teilmengen eines genetischen Materials.

Teilmengen innerhalb eines genetischen Materials sind zum Beispiel Chromosomen innerhalb eines Genoms. In diesem Fall stellt ein Chromosom eine Teilmenge aus einem Genom dar, das mehrere unterschiedliche Chromosomen enthält. Abgrenzbare Teilmengen können aber auch Deletionen bzw. Insertionen innerhalb eines einzelnen Chromosoms sein. In diesem Fall stellen die Deletionen bzw. Insertionen die abgrenzbaren Teilmengen dar und das einzelne Chromosom das genetische Material. Genetische Informationen eines Chromosoms sind z.B. Zielsequenzen, die lediglich auf diesem Chromosom vorkommen können, also für dieses Chromosom spezifisch sind.

Ein System, in dem die abgrenzbaren Teilmengen unterschiedliche Chromosomen eines Genoms darstellen, ist z.B. ein Polkörperchen.

Polkörperchen entstehen in Vertebraten bei der Bildung und Reifung von Eizellen, welche zur Vermehrung der jeweiligen Art benötigt werden.

Beim Menschen kann kinderlosen Paaren oder Frauen zur Erfüllung ihres Kinderwunsches eine assistierte Reproduktion angeboten werden. Hierzu stehen derzeit mehrere Verfahren zur Verfügung, die zu einer durchschnittlichen Schwangerschaftsrate von ca. 10% je Eizelle führen. Indirekte, über die Polkörperchen der Eizelle durchgeführte Analysen haben gezeigt, dass Eizellen in einem hohen Prozentsatz eine Fehlverteilung für einzelne Chromosomen aufweisen. Diese Aneuploidien führen zu nicht lebensfähigen Embryonen und begründen vermutlich die niedrige Implantationsrate nach assistierter Reproduktion.

In der Bevölkerung sind ca. 10% aller Paare ungewollt kinderlos. Die Gründe für die Unfruchtbarkeit finden sich zum einen in organischen Defekten bei der Frau oder beim Mann begründet, sie kann aber auch genetische Ursachen haben. Für bis zu 70% der Fehlgeburten können genetische Gründe, zumeist chromosomale Fehlverteilungen verantwortlich gemacht werden (Griffin 1996).

Diese chromosomalen Aneuploidien resultieren zumeist aus einer gestörten Oogenese (Angell 1993). Bei der Spermiogenese werden nur in 2-4% aneuploide Zellen gefunden (Zenzes 1992). Diese und andere Ursachen führen dazu, dass unter natürlichen Bedingungen ein hoher Prozentsatz aller befruchteten Eizellen nicht zu einer intakten Schwangerschaft führt.

Wie oben bereits angeführt, kann kinderlosen Paaren oder Frauen eine in vitro Fertilisation oder eine andere assistierte Reproduktionstechnik, wie z.B. die intrazytoplasmatische Spermieninjektion (ICSI), angeboten werden, wobei mit den derzeit zur Verfügung stehenden in vitro Fertilisationsmethoden eine durchschnittliche Schwangerschaftsrate von 10% je Eizelle erzielt werden kann. Die Auswertung großer Studien hat gezeigt, dass die Schwangerschaftsrate bei Frauen über dem 35. Lebensjahr deutlich abnimmt und bei Frauen über 40 Jahren unter 10% liegt. Dies geht mit der Beobachtung einher, dass Mütter ab dem 35. Lebensjahr ein erhöhtes Risiko für ein Kind mit chromosomaler Fehlverteilung tragen.

In der pränatalen Diagnostik werden nur diejenigen Kinder mit einer Chromosomen-Fehlverteilung diagnostiziert, die zumindest bis zum Zeitpunkt der Diagnostik lebensfähig sind. So findet man z.B. zum Zeitpunkt der Amniozentese mehr Kinder mit Trisomien oder Monosomien als zum Zeitpunkt der Geburt, da viele dieser Kinder im Verlauf der Schwangerschaft versterben.

Betrachtet man nur die Trisomien, so sind im wesentlichen nur Kinder mit einer Trisomie 21 oder mit einem überzähligen oder fehlenden X- bzw. Y-Chromosom lebensfähig. Die oben genannten geringen Implantationsraten könnten durch Aneuploidien der Eizellen auch für andere Chromosomen begründet sein, die entweder zu keiner Implantation oder zu einem sehr frühen Abort führen. Dies wird gestützt durch Chromosomenanalysen an Abortmaterial.

Während der Eizellreifung muss die initial diploide Eizelle ihren Chromosomensatz reduzieren. Dieser Vorgang vollzieht sich in der ersten und zweiten Reifeteilung. In der ersten Reifeteilung (I. Reduktionsteilung) werden die homologen Chromosomen getrennt. In der zweiten Reifeteilung erfolgt die Trennung der Chromatiden. Das genetische Material der dabei entstehenden Tochterzellen wird jeweils in Form der Polkörperchen in den perivittelinen Spalt der Eizelle transferiert. Polkörperchen entsprechen in ihrem Aufbau einer Zelle, haben jedoch einen nur minimalen Zytoplasmaanteil. Das erste Polkörperchen entsteht während der Ovulation, das zweite Polkörperchen wird 3-4 h nach der Penetration des Spermiums in die Eizelle ausgeschleust. Die beiden Polkörperchen unterscheiden sich in der Menge ihres genetischen Materials. Das erste Polkörperchen enthält 23 Chromosomen mit zwei Chromatiden (2n), während das zweite wie auch die reife Eizelle nur 23 einfache Chromosomen mit nur einem Chromatid enthält (1n). Die Polkörperchen haben keinerlei Funktion und werden in der frühen Embryonalentwicklung resorbiert. Eine biologische Signifikanz des Polkörperchens für das Embryo ist nicht bekannt (siehe Abstract zu "Preimplantation Genetic Diagnosis, Polar Body Biopsy" von The First World Congress On: Controversies in Ostetrics, Gynecology & Infertility, Prague, Czech Republic - 1999 von Y. Verlinsky, A. Kuliew und Flyer zu Polkörperchendiagnostik der Pränatal-Medizin München, Dr. med Karl-Philip Gloning et al. Im Abstract zu "Preimplantation Genetic Diagnosis, Polar Body Biopsy" von The First World Congress On: Controversies in Ostetrics, Gynecology & Infertility, Prague, Czech Republic - 1999 von Y. Verlinsky, A. Kuliew ist die Untersuchung erster und zweiter Polkörperchen mittels "sequential testing", was eine Entnahme des Polkörperchens voraussetzt offenbart. Aus 179 erfolgreichen künstlichen Schwangerschaften resultierten 135 gesunde Kinder, die keine Schäden durch diesen Eingriff erlitten haben.

M. Montag, K. van der Ven, H. van der Ven *"Erste klinische Erfahrungen mit der Polkörperchendiagnostik in Deutschland")* berichtet über erste Erfahrungen mit der Polkörperchendiagnostik in Deutschland, wonach die Schwangerschaftsraten unter Einbeziehung von Polkörperchen-Diagnostik erfreulich hoch sind.

"Einführung in die Präimplantationsdiagnostik", E. Schwinger, Lübeck, Quelle: http://www.studgen.uni-mainz.de/manuskripte/schwinger.pdf. beschreibt, dass eine Erhöhung der Fehlbildungsrate nach PID mittels Polkörperchen- oder Blastozystenanalyse nicht feststellbar ist. Das Dokument verdeutlicht die engen Zeitfenster, die bei einer Präfertilisationsdiagnostik (PFD) zur Verfügung stehen.

Gemäß einem Flyer zu Polkörperchendiagnostik der Pränatal-Medizin München, Dr. med Karl-Philip Gloning et al. sprechen die bisher veröffentlichten Daten dafür, dass eine Polkörperentnahme nicht mit einer nennenswerten Erhöhung des allgemeinen Basisrisikos von 2 - 4 % für Entwicklungsstörungen verbunden ist.

Es existieren also bereits menschliche Individuen, die aus Eizellen hervorgegangen sind, denen das erste Polkörperchen entfernt wurde, die keine Schäden durch diesen Eingriff erlitten haben. Damit bietet sich die Polkörperchenanalyse als Methode der Wahl an, wenn es darum geht, Eizellen auf Ihre Eignung für eine erfolgreiche Befruchtung hin zu untersuchen.

Die Polkörperchen repräsentieren die Anzahl der Chromosomen in der Eizelle und stehen für die Durchführung einer genetischen Analyse zur Verfügung.

Die Einzelzelldiagnostik an einem Polkörperchen setzt dessen sterile Entnahme aus dem perivittelinen Spalt der Eizelle voraus. Die klassische Entnahmetechnik ist die mittels eines Mikromanipulators unterstützte Eröffnung der Zona Pellucida und anschließende Isolierung des Polkörperchens.

Durch die Möglichkeit der genauen Beobachtung des Fertilisierungsvorgangs während der In-Vitro-Fertilisation (IVF) hat sich gezeigt, dass sich ein Teil der Eizellen nicht fertilisieren lässt oder dass sich bereits fertilisierte Eizellen nicht weiter teilen. Viele dieser frustranen Fertilisierungsversuche sind vermutlich durch Aneuploidien der Eizellen begründet. Mehrere Arbeitsgruppen haben sich mit der genetischen Analyse von Polkörperchen beschäftigt. Für diese Studien wurden die Polkörperchen isoliert und einer Fluoreszenz in situ Hybridisierung (FISH) unterzogen. Bei dieser Analyse werden mit einem Fluoreszenzfarbstoff markierte, für bestimmte Chromosomen spezifische Moleküle auf die Chromosomen der Polkörperchen hybridisiert. Findet sich hier für ein Chromosom eine abweichende Anzahl an Signalen, lässt dies auf eine aberrante Chromosomenverteilung während der Oogenese schließen. Mit Hilfe dieser Methode konnte anhand der Analyse von 3943 Oozyten im Jahre 1999 gezeigt werden, dass 43% der Oozyten eine chromosomale Fehlverteilung aufwiesen, wobei die Fehlverteilungen sowohl in der ersten: als auch in der zweiten Reifeteilung aufgetreten sind. Bei dieser Studie sind nur die Chromosomen 13, 18 und 21 hinsichtlich ihrer korrekten Verteilung analysiert worden (Verlinsky 1998). Eine Verfeinerung der Technik erlaubt heute die gleichzeitige Analyse von 5 verschiedenen Chromosomen. Die Methode ist schon im Ansatz in der Anzahl der analysierbaren Chromosomen limitiert, da für jedes Chromosom ein anderes Fluorochrom verwendet werden muss und eine eindeutige Auswertung nur dann möglich ist, wenn sich die Signale nicht überlagern.

Aus der US 6,143,564 ist ein Verfahren zur Variation der genetischen Information der Eizelle von Tieren unter Zuhilfenahme von Polkörperchen bekannt.

Aus der JP 2086800 ist ein Verfahren zum Nachweis über das Vorhandensein eines bestimmtes Genes in einer befruchteten Eizelle bekannt, bei dem das erste und das zweite Polkörperchen untersucht werden.

Chromosomenspezifische Fänger-Moleküle für In-situ-Hybridisierungen im Wege des FISH-Verfahrens sind aus US 5,817,462 bekannt. Durch verschiedene Kombinationen verschiedener Fluorophore können dabei alle menschlichen Chromosomen simultan nachgewiesen werden. Bei höheren Chromosomenzahlen wird die Zahl der erforderlichen Kombinationen von geeigneten Fluorophoren immer komplexer, ebenso wie deren Analyse. Gegebenenfalls müssen dann einzelne Chromosomen unter dem Mikroskop an Hand ihrer Größe unterschieden werden.

FISH-Experimente sind also nur in begrenztem Umfang geeignet, die Chromosomen innerhalb eines genetischen Materials simultan zu quantifizieren. Die Quantifizierung abgrenzbarer Teilmengen eines genetischen Materials mittels derartiger Verfahren ist zunächst auf Chromosomen beschränkt. Die Anzahl der kombinierbaren Farben ist begrenzt, und im Falle einer Polkörperchenuntersuchung ist das Polkörperchen nach Durchführung einer FISH-Analyse verbraucht. Eine zuverlässige Aussage über die Integrität eines gesamten Chromosomensatzes zu treffen ist mit dem genannten Verfahren bisher unmöglich. Dies liegt auch darin begründet, dass die Vorbereitung zu FISH-Hybridisierungen beim Polkörperchen nicht so durchgeführt werden können wie im Falle der etablierten FISH-Verfahren an Metaphase-Zellen, weil sich die genomische DNA eines Polkörperchens nicht weiter teilen lässt, und weil sich die Chromosomen eines Polkörperchens nicht wie herkömmliche Chromosomen kondensieren lassen.

Auch neben der FISH-Technik angewandte Techniken der Chromosomenbänderung oder der konventionellen Homogenfärbung haben sich nicht als befriedigend erwiesen, da sich je nach angewandter Technik in verschiedenen Studien unterschiedlichste Anomalieraten ergeben haben (Eckel et.al. 2001).

Aus der US 6,060,251 ist ein Verfahren zur Bestimmung der chromosomalen Identität einer Probe, die genomische DNA enthält, bekannt, wobei genomische DNA amplifiziert und mit Markierungsmitteln versehen wird. Das beschriebene Amplifikationsverfahren ist ein unspezifisches Amplifikationsverfahren, bei dem repetitive Sequenzen als Primerbindungsstellen dienen. Das Amplifikationsprodukt wird dann mittels einer DNA-Bibliothek untersucht. Der Nachweis erfolgt über die Detektion von Hybriden, wobei die Fänger-Moleküle aus der DNA-Bibliothek auf einen festen Träger aufgebracht sein können.

Grundsätzlich besteht die Möglichkeit, eine Aussage über das Vorhandenseins eines Chromosoms in einer Probe zu treffen, indem man im Rahmen einer spezifischen Amplifikation eine Zielsequenz amplifiziert, die nur auf einem bestimmten Chromosom vorkommt, und diese Zielsequenz mittels der Bildung eines Fänger-Zielsequenz-Hybrides nachweist. Um solche Fänger-Zielsequenz-Hybride nachweisen zu können, ist eine Mindestmenge von diesen erforderlich, und eine entsprechende Mindestmenge an Kopien der Zielsequenz muss generiert werden, weshalb eine Amplifikation erforderlich ist. Da im Rahmen einer spezifischen Amplifikation in der Regel nur Kopien von einer bestimmten Zielsequenz erzeugt werden, lässt sich aus dem Nachweis der entsprechenden Hybride nur über das Vorhandensein von Chromosomen rückschließen, die diese Zielsequenz aufweisen. Aus der WO 00/24925 sind Verfahren und Mittel zur Ermittlung der chromosomalen Zusammensetzung einer Zelle bekannt, bei denen das zu untersuchende Erbmaterial zunächst mittels einer unspezifischen PCR-Amplifikation amplifiziert wird, wobei Polkörperchen ebenfalls als Quelle für derartiges Erbmaterial genannt sind. Aufgeführt sind folgenden PCR-Techniken: DOP-PCR, Primer extension preamplification PCR, ligation mediated PCR, tagged PCR, und Alu-PCR. Bei diesen Amplifikationsverfahren ist durch äußerst unspezifische Primer sichergestellt, dass ein repräsentativer Querschnitt des in einer Zelle vorhandenen genetischen Materials amplifiziert wird. Neben Zielsequenzen, die einzelnen Chromosomen zugeordnet werden können, kommt darin eine Vielzahl von völlig unspezifischen Sequenzen vor. Das Amplifikationsprodukt kann mit einem Genchip untersucht werden. Mit dem beschriebenen Verfahren soll es möglich sein, chromosomale Differenzen und Aneuploidien nachzuweisen. Dabei wird neben der zu untersuchenden Probe eine Referenzprobe parallel amplifiziert, beide Proben werden mit unterschiedlichen Markierungsagenzien versehen und auf einen Chip aufgebracht, der Fängermoleküle aufweist, die mit den jeweiligen Zielsequenzen Hybride bilden können. Wegen der Vielzahl koamplifizierter unspezifischer Sequenzen ergeben sich allerdings folgende Probleme:
- die Zielsequenzen, auf deren Nachweis es letztendlich ankommt, liegen in einem Gemisch mit einer Vielzahl völlig unspezifischer Sequenzen verdünnt vor,
- die Vielzahl koamplifizierter unspezifischer Sequenzen kann Sequenzen umfassen, die den Zielsequenzen so ähnlich sind, dass die Fänger-Moleküle Hybride ausbilden, die bei einer Detektion dahingehend interpretiert werden, dass die dem jeweiligen Fängermolekül zugeordnete Zielsequenz vorhanden ist, was der Wirklichkeit dann nicht oder nur bedingt entspricht,
- durch die Verdünnung von Zielsequenzen innerhalb einer Vielzahl unspezifischer Sequenzen, die direkt auf die Unspezifität des Amplifikationsverfahrens zurückgeht, ist eine höhere Zyklenzahl erforderlich, um eine Mindestmenge an Zielsequenzen zu erzeugen, die zu einer detektierbaren Mindestmenge an Fänger-Zielsequenz-Hybriden führen kann; mit jedem Zyklus erhöht sich aber auch die Gefahr, dass Amplifikationsprodukte mit Fehlstellen entstehen, was wiederum zu Schwierigkeiten bei der Bildung der erwünschten "richtigen" Fänger-Zielsequenz-Hybride und ihrer Unterscheidung von unerwünschten Fehlhybriden führt,
- hohe Zyklenzahlen führen dazu, dass die Bildung von PCR-Produkten, die auf eine Zielsequenz zurückgehen, ab einem bestimmten Zyklus nicht mehr exponentiell zunimmt, sondern stagniert. Ab welchem Zyklus dies geschieht, ist unter anderem abhängig von der Anfangskonzentration dieser Zielsequenz. Daher beginnen die Konzentrationszunahmen verschiedener amplifizierter Zielsequenzen eines genetischen Materials bei gleichzeitiger Amplifikation mittels PCR und unterschiedlichen Anfangskonzentrationen der Zielsequenzen in jeweils unterschiedlichen Stadien der Amplifikation zu stagnieren. Ein Aussage über die relative Quantität dieser Zielsequenzen kann dann nicht mehr getroffen werden.

Nach der WO 00/24925 kann das bei der unspezifischen Amplifikation erhaltene Produkt einer nachfolgenden spezifischen Amplifikation unterworfen werden, um eine Aussage über das Vorhandensein der Zielsequenz der spezifischen Amplifikation im Ursprungsmaterial zu treffen. Mit einer spezifischen Amplifikation wird nur eine ganz bestimmte Zielsequenz amplifiziert. Mit dem Produkt der zweiten Amplifiktion kann daher nur eine Aussage über das Vorhandensein der darin amplifizierten Zielsequenz getroffen werden. Die relative Quantität von Produkten unterschiedlicher zweiter Amplifikationsexperimente zu vergleichen führt bereits wegen Unwägbarkeiten in der vorhergehenden, unspezifischen Amplifikation zu keiner brauchbaren Aussage - hinzu kommt, dass jedes Amplifikationsexperiment für sich genommen von einer Vielzahl von Parametern beeinflusst wird, die schwer zu reproduzieren sind. Notgedrungen schwanken daher die Ergebnisse dieser kombinierten Amplifikationsverfahren. Eine simultane Analyse derartiger Zielsequenzen unter möglichst vergleichbaren Bedingungen ist unter Verwendung von aus der WO 00/24925 bekannten Verfahren deshalb nicht möglich. Um eine konkrete Aussage über das Vorhandensein einer Zielsequenz einer nach der WO 00/24925 untersuchten Zelle zu treffen ist außerdem eine umständliche, material- und zeitaufwändige Vorgehensweise erforderlich.

In der Datenbank PubMed bei NCBI, Adresse www.ncbi.nlm.nih.gov, Zusammenfassung zu Rapid detection of common autosomal aneuploidies by quantitative flourescent PCR on uncultured amniocytes. RAHIL, H. u.a., Eur. J.Hum. Genet. (August 2002) 10(8) 462-6 ist eine Koamplifikation von DSCR1, DCC, und RB1 beschrieben, wobei für jedes dieser Gene ein eigenes Primerpaar benötigt wird, für die gesamte Koamplifikation folglich drei Primerpaare für die drei Genregionen.

Aus WO 02/29066 A ist ein Primer bekannt, der in ERS-PCR eingesetzt worden ist, um bestimmte DNA-Fragmente zu isolieren.

Datenbank PubMed bei NCBI, Adresse www.ncbi.nlm.nih.gov, Zusammenfassung zu: Identification of chromosomal translocations in leukemias by hybridization with oligonucleotide microarrays, NASEDKINA, T. u.a. Haematologica (April 2002) 87 (4) 363-72 und Datenbank PubMed bei NCBI, Adresse www.ncbi.nlm.nih.gov, Zusammenfassung zu: DNA microarray technology for neonatal screening. DOBROWOLSKI, S.F. u.a., Acta Paediatr. Suppl. (1999) 88 (432) 61-4 beschreiben Multiplex-PCR-Reaktionen. Dabei werden mehrere unterschiedliche Sequenzen zugleich amplifiziert.

WO 02/44411 beschreibt Verfahren zur Feststellung von Aneuploidien, das auf Expression-Profiling basiert. Es wird hierbei die Expression von Genen identifiziert, die auf einem Chromosom vorkommen, und daraus auf das Chromosom geschlossen.

Verfahren, die unter Zuhilfenahme von Chromosomenspreizung durchgeführt werden, sind aus WO 00/24925 bekannt.

EP 1 026 260 A1 beschreibt die Analyse von Gewebeproben und mRNA, folglich von Material aus einer Vielzahl von Zellen.

Amstrong et al. (Cytometry, 2000, Band 40, Seiten 202 ff.) beschreiben die Bestimmung der Häufigkeit von Allelen eines Gens mittels PCR und allelspezifische Proben, die an Partikel gebunden sind. Die Proben sind 100 % homolog zur Zielsequenz. Die Primer binden allerdings nicht an mehrere Bindungsstellen im genetischen Material.

DE 101 02 678 A1 und DE 100 59 776 A1 befassen sich mit dem Nachweis von Aneuploidien, wobei die beschriebenen Methoden nicht brauchbar sind, Aneuploidien ausgehend von einer einzigen Zelle oder gar einem Polkörperchen nachzuweisen.

McCarthey et al. (Proceedings of The National Academy of Sciences of the United States, Band 92, Nr.12, 1995, 5302 ff.) beschreibt die Kartierung von Genomen (RH-Mapping). Der Zweck des RH-Mappings ist die physikalische Kartierung einzelner Loci, aber nicht die Quantifizierung von genetischem Material.

Aus der US 6,329,140 werden Grundlagen und Möglichkeiten der DNA-Chip-Technologie im Zusammenhang mit Verfahren zur Auswahl geklonter Organismen erläutert.

Aus der EP 1 026 260 ist ein Verfahren zur gleichzeitigen Ermittlung der Genexpression und genetischer Abnormalitäten unter Verwendung von DNA-Arrays bekannt, wobei der beschriebene DNA-Array zur Genexpresssionsanalyse und zur Feststellung chromosomaler Abnormalitäten in einer Gewebeprobe geeignet ist. Auf dem Chip sind hierzu Fänger-Moleküle vorgesehen, die bestimmten Chromosomen zugeordnet werden können. Mit dem Verfahren und dem beschriebenen DNA-Chip werden exprimiertes und nicht exprimiertes Probenmaterial voneinander unterschieden.

Allgemein lässt sich sagen, dass eine Erweiterung der technischen Hilfsmittel zur Durchführung von Analysen über das Vorhandensein abgrenzbarer Teilmengen und deren relative Quantität innerhalb eines genetischen Materials, insbesondere von Chromosomenanalysen an Polkörperchen begrüßenswert ist.

Gemäss dem Embryonenschutzgesetz vom 13.12.1990 ist in Deutschland die Durchführung einer Präimplantationsdiagnostik zum Nachweis chromosomaler Anomalien an menschlichen Embryonen verboten und deren Selektion nicht möglich. Damit entfällt die Analyse des zweiten Polkörperchens bei menschlichen Eizellen. Bei Entstehung des ersten Polkörperchens ist die Eizelle aber noch nicht befruchtet, und solange keine Befruchtung stattgefunden hat, ist die Eizelle nicht Gegenstand des Embryonenschutzgesetzes. Daher könnte eine Verbesserung der Schwangerschaftsraten durch die zytogenetische Analyse des 1. Polkörperchens erreicht werden. Dabei würde eine Erkennung aneuploider Oozyten vor der Befruchtung ermöglicht und diese könnten aus dem weiteren Fertilisationsverfahren ausgeschlossen werden (Eckel et al. 2001). Dafür steht nur eine begrenzte Zeit zur Verfügung, innerhalb derer die Eizelle mit Erfolg befruchtet werden kann. Diese Zeitspanne bewegt sich in einer Größenordnung von 1-2 Tagen.

Eine derartige Methodik könnte sich auch bei der Reproduktion anderer Vertebraten als nützlich erweisen, z.B. bei der Reproduktion vom Aussterben bedrohter Tierarten. In solchen Fällen wäre eine Analyse auch des 2. Polkörperchens nicht grundsätzlich verboten. Bei Entnahme des zweiten Polkörperchens ist aber die zur Verfügung stehende Zeitspanne vor Implantation der befruchteten Zelle in der Regel deutlich niedriger als die nach Entnahme des 1. Polkörperchens zur Verfügung stehende Zeit.

Da Eizellen nur innerhalb einer kurzen Zeitspanne erfolgreich befruchtet werden können, sollte das Verfahren schnell sein und eine möglichst zuverlässige Aussage über die relative Quantität der einzelnen Chromosomen zulassen.

Aufgabe der Erfindung ist es, ein Verfahren zur Bestimmung der relativen Häufigkeit abgrenzbarer Teilmengen eines genetischen Materials zur Verfügung zu stellen, das in geringster Menge vorliegt, und das geeignet ist, die in einem Polkörperchen vorhandenen Chromosomen und ihre relative Quantität zueinander nachzuweisen.

Die Aufgabe wird durch ein Verfahren mit den Merkmalen des Anspruchs 1 gelöst. Vorteilhafte Ausgestaltungen hiervon sind in den weiteren, hiervon abhängigen Ansprüchen angegeben.

Die Aufgabe wird gelöst durch ein Verfahren zur Bestimmung der relativen Häufigkeit abgrenzbarer Teilmengen eines genetischen Materials, wobei das genetische Material mittels Polymerase-Ketten-Reaktion amplifiziert wird, bei dem Primer verwendet werden, die komplementär zu Primerbindungsstellen sind, die in dem genetischen Material an mehreren Stellen vorhanden sind, und die jeweils benachbart zu einer für jeweils eine Teilmenge spezifischen Zielsequenz mit vorbestimmter Länge sind, so dass ein Amplifikationsprodukt erhalten wird, das im wesentlichen nur amplifizierte Sequenzen aufweist, die eine für die jeweilige genetische Information spezifische Zielsequenz mit vorbestimmter Länge umfassen, die zur Detektion mittels Hybridisierung geeignet sind, wobei das Amplifikationsprodukt mittels eines Hybridisierungsexperiments zur Bestimmung der relativen Häufigkeit der abgrenzbaren Teilmengen analysiert wird.

Es werden Zielsequenzen gleichzeitig amplifiziert, die unterschiedlich sind, und die jeweils für eine Teilmenge des genetischen Materials spezifisch sind. Diese Zielsequenzen werden alle unter den gleichen Reaktionsbedingungen amplifiziert. Sie liegen in dem Produkt des Amplifikationsverfahrens in einer wesentlich höheren Konzentration vor als in unspezifischen Amplifikationsverfahren nach dem Stand der Technik. Hierdurch sind geringere Zyklenzahlen als nach dem Stand der Technik erforderlich, um mittels Hybridisierung nachweisbare Mengen an Zielsequenzen zu erzeugen. Dies geht einher mit einer verringerten Fehlerrate bei der Amplifikation, und mit einer geringeren Anzahl von Fehlhybridisierungen im Falle einer Detektion. Das Produkt des Verfahrens gestattet also schnellere, sicherere und aussagekräftigere Analysen durchzuführen als mit Produkten von bekannten Amplifikationsverfahren, bei denen viele unterschiedliche Sequenzen zugleich amplifiziert werden.

Das mit dem Verfahren hergestellte Amplifikationsprodukt enthält im wesentlichen nur amplifizierte Sequenzen, die eine für die jeweilige genetische Information spezifische Zielsequenz mit vorbestimmter Länge umfassen, die zur Detektion mittels Hybridisierung geeignet ist. Im wesentlichen kann mindestens 80%, vorzugsweise 90% oder 95%, spezifische Zielsequenzen bedeuten.

Für eine erfolgreiche quantitative Detektion ist es aus statistischen Gründen zweckmäßig, dass die Primerbindungsstellen des bzw. der verwendeten Primer zu mindestens 10, 20, 30, 50 oder 100 spezifischen Zielsequenzen einer abgrenzbaren Teilmenge benachbart angeordnet sind, so dass mindestens 10, 20, 30, 50 oder 100 spezifischen Zielsequenzen pro abgrenzbarer Teilmenge amplifiziert werden.

Die Aufgabe wird ferner gelöst durch ein Verfahren mit den Schritten:
- Durchführen eines Verfahrens zur Amplifikation genetischer Informationen aus genetischem Material, das mehrere voneinander abgrenzbare Teilmengen genetischen Materials umfasst, so dass ein Amplifikationsprodukt erhalten wird, dessen Sequenzen Zielsequenzen umfassen, die den abgrenzbaren Teilmengen zugeordnet werden können, und das zur Detektion mittels Hybridisierung geeignet ist,
- Vermengen des Amplifikationsprodukts mit Fänger-Molekülen auf einem DNA-Chip, so dass sich Hybride aus Fänger-Molekülen und Teilen des Amplifikationsprodukts ausbilden, wobei der DNA-Chip zumindest zwei Gruppen von Spots umfasst, wobei die Spots innerhalb einer Gruppe unterschiedliche Fänger-Moleküle aufweisen und eine Gruppe von Spots jeweils einer der abgrenzbaren Teilmengen des genetischen Materials zugeordnet ist,
- quantitative Detektion der jeweils in einem Spot mit unterschiedlichen Fänger-Molekülen des DNA-Chips gebildeten Hybride, so dass für jeden Spot jeweils ein Detektionswert erhalten wird,
- Mittelwertbildung der Detektionswerte der auf dem DNA-Chip vorhandenen Gruppen von Spots,
- Bestimmung der relativen Häufigkeit von Teilmengen genetischen Materials innerhalb eines genetischen Materials durch Vergleich der Mittelwerte.

Ein derartiges erfindungsgemäßes Verfahren unter Verwendung eines DNA-Chips gestattet eine Mittelwertbildung der Detektionswerte von Signalen, die abgrenzbaren Teilmengen des genetischen Materials zugeordnet werden können, und hat den Vorteil, dass das Verfahren eine hohe Toleranz gegenüber der Verstärkung sonst nachteilhafter und mit dem Amplifikationsverfahren einhergehender Effekte aufweist.

Bei einem erfindungsgemäßen Verfahren, bei dem die Homologie zwischen den Primern und den jeweiligen Primerbindungsstellen in einem Bereich von 80%-100%, und vorzugsweise in einem Bereich von 90%-100% liegt, ist der Anteil an Zielsequenzen im Amplifikationsprodukt besonders hoch.

Bei einem Verfahren, in dem der Abstand zwischen Primerbindungsstellen und den benachbarten spezifischen Zielsequenzen nicht mehr als 1000, vorzugsweise nicht mehr als 300, und insbesondere nicht mehr als 100 Basen beträgt, ist die für die Durchführung des Verfahrens benötigte Zeit gering und der Anteil an Zielsequenzen im Amplifikationsprodukt besonders hoch.

Bei einem Verfahren, in dem die vorbestimmten Längen der spezifischen Zielsequenzen zwischen 15 und 80 Basen, und bevorzugt zwischen 20 und 50 Basen betragen, ist die Spezifität der Zielsequenzen für die jeweilige abgrenzbare Teilmenge besonders gut gewährleistbar.

Bei einem Verfahren, in dem alle spezifischen Zielsequenzen im wesentlichen gleiche Längen aufweisen, erhält man einen Pool von Zielsequenzen, die mit zu deren Nachweis vorgesehenen Fänger-Oligonukleotiden Hybride bilden, die sehr ähnliche Eigenschaften aufweisen. Z.B. haben die Hybride ähnliche Schmelztemperaturen, wenn sie gleich lang sind, d.h. sie sind ähnlich stabil, und z.B. läuft die Bildung derartiger Hybride mit vergleichbaren Geschwindigkeiten ab.

Bei einem erfindungsgemäßen Verfahren, in dem im Verlauf der Polymerase-Ketten-Reaktion mit Markierungen versehene Nukleotidbausteine verwendet werden, wird ein Amplifikationsprodukt erhalten, das nach Hybridisierung der Zielsequenzen mit entsprechenden Fänger-Oligonukleotiden leicht anhand der jeweils eingebauten Markierung nachzuweisen ist.

Bei einem erfindungsgemäßen Verfahren, in dem außerdem die Schritte
- Vermengen des Amplifikationsprodukts mit Fänger-Molekülen, so dass sich Hybride aus Fänger-Molekülen und Zielsequenzen ausbilden, und
- Detektion der Hybride,
durchgeführt werden, kann anhand der Art der detektierten Hybride und anhand deren vorhandener Menge eine Aussage über die Menge und das Vorhandensein der jeweiligen abgrenzbaren Teilmenge in dem genetischen Material getroffen werden.

Bei einem erfindungsgemäßen Verfahren, in dem außerdem die Schritte
- Vermengen des Amplifikationsprodukts mit Fänger-Molekülen, so dass sich Hybride aus Fänger-Molekülen und Zielsequenzen ausbilden, und
- Detektion der Hybride durchgeführt werden,
und bei dem die Fänger-Moleküle auf einem DNA-Chip angeordnet sind, können alle gebildeten Hybride zugleich detektiert und auf engstem Raum miteinander verglichen werden.

Bei einem erfindungsgemäßen Verfahren, in dem außerdem die Schritte
- Vermengen des Amplifikationsprodukts mit Fänger-Molekülen, so dass sich Hybride aus Fänger-Molekülen und den Zielsequenzen ausbilden, und
- Detektion der Hybride, durchgeführt werden und
bei dem die Fänger-Moleküle aus Oligonukleotiden ausgebildet sind, kann die Genauigkeit der Hybridisierung gut gewährleistet werden.

Bei einem erfindungsgemäßen Verfahren, in dem ein DNA-Chip verwendet wird, bei dem in einem einzelnen Spot jeweils gleiche Fänger-Moleküle vorgesehen sind, kann anhand der Intensität der Detektion innerhalb dieses Spots eine Aussage über das Vorhandensein einer bestimmten Zielsequenz innerhalb des Amplifikationsprodukts getroffen werden.

Bei einem erfindungsgemäßen Verfahren, in dem ein DNA-Chip verwendet wird, bei dem in einem einzelnen Spot unterschiedliche Fänger-Moleküle für unterschiedliche Zielsequenzen vorgesehen sind, die alle einer der abgrenzbaren Teilmengen des genetischen Materials zugeordnet sind, genügt die Messung der Intensität in einem solchen Spot:
- um eine Aussage über das Vorhandensein dieser abgrenzbaren Teilmenge im genetischen Material treffen zu können, und
- um im Vergleich mit der Intensität der anderen Spots eine zuverlässige Aussage über die relative Quantität der abgrenzbaren Teilmenge im genetischen Material treffen zu können.

Ein erfindungsgemäßes Verfahren, bei dem das genetische Material aus einer Einzelzelle stammt oder auf diese zurückzuführen ist, gestattet eine schnelle, sichere und qualitativ hochwertige Aussage über die Quantität abgrenzbarer Teilmengen innerhalb des genetischen Materials der Einzelzelle zu.

Ein erfindungsgemäßes Verfahren, bei dem das genetische Material ein Chromosomensatz aus einem Polkörperchen einer Eizelle ist, gestattet eine rasche und qualitativ hochwertige Aussage über die Eignung der Eizelle für eine Befruchtung, ohne dass die Eizelle selbst geschädigt wird.

Ein erfindungsgemäßes Verfahren, bei dem eine abgrenzbare Teilmenge aus einem oder mehreren Chromosomen besteht, gestattet eine Aussage über die Integrität der chromosomalen Zusammensetzung eines genetischen Materials.

Ein erfindungsgemäßes Verfahren, bei dem eine abgrenzbare Teilmengen aus einem oder mehreren Genen besteht, gestattet eine Aussage über das Vorhandensein von Deletionen oder Insertionen innerhalb eines genetischen Materials.

Ein erfindungsgemäßes Verfahren, bei dem ein genetisches Referenzmaterial unter ansonsten gleichen Reaktionsbedingungen parallel amplifiziert wird, stellt ein Amplifikationsprodukt zur Verfügung, das es einerseits gestattet, die abgrenzbaren Teilmengen eines genetischen Materials und ihre relative Quantität zueinander zu bestimmen, und das es zusätzlich gestattet, eine Verifikation dieser Quantifizierung durchzuführen.

Das erfindungsgemäße Verfahren weist Primer auf, die gegenüber Primerbindungsstellen komplementär sind, die an mehreren Stellen innerhalb eines genetischen Materials vorkommen, und die jeweils benachbart zu einer für jeweils eine Teilmenge spezifischen Zielsequenz sind. Das bedeutet, dass identische oder im wesentlichen identische Primerbindungsstellen benachbart sind zu unterschiedlichen Zielsequenzen, was wiederum impliziert, dass ein einzelner Primer oder ein einzelnes Primerpaar in der Lage ist, mehrere, unterschiedliche spezifische Zielsequenzen zu amplifizieren.

Im Vergleich zu Multiplex-PCR-Reaktionen ist es ein wesentliches Merkmal des erfindungsgemäßen Verfahrens, dass dabei ein Amplifikationsprodukt erhalten wird, das im wesentlichen nur amplifizierte Sequenzen aufweist, die eine für die jeweilige genetische Information spezifische Zielsequenz mit vorbestimmter Länge umfassen, die zur Detektion mittels Hybridisierung geeignet sind.

Klassische Koamplifikationen unterschiedlicher Primer oder konventionelle Multiplex-PCR-Experimente gehen von großen Materialmengen, etwa fötaler DNA, Zellkulturen oder vom Blut Neugeborener. Expression-Profiling-Verfahren gehen von mRNA aus, mRNA stellt eine kleine Auswahl dessen dar, was auf einer genomischen DNA enthalten ist. Es handelt sich um ein ganz anderes Ausgangsmaterial als es etwa die chromosomale DNA eines Polkörperchens. Das Polkörperchen ist nicht transkriptionsaktiv und enthält dementsprechend keine mRNA. Alle Methoden, die auf der Quantifizierung von mRNA beruhen, sind zur Polkörperchen-Untersuchung nicht geeignet. Bekannte Verfahren sind daher insbesondere zur Detektion von Chromosomenanomalien einer einzigen Zelle nicht geeignet. Diese Leistung erbringt hingegen das erfindungsgemäße Verfahren.

Dem Grunde nach sind zwar alle Nukleinsäuren zur Detektion mittels Hybridisierung geeignet. Dabei wird allerdings vorausgesetzt, dass eine für den Vorgang der Detektion selbst ausreichende Menge an Material vorhanden ist.

Ein einzelnes oder einige wenige Moleküle einer Zielnukleinsäure, die an einen Fänger gebunden sind, reicht hierfür jedenfalls nicht aus. Darum muss das Probenmaterial, wenn es sich um eine Menge unterhalb der Nachweisgrenze handelt, amplifiziert werden.

Das erfindungsgemäße Verfahren ermöglicht eine quantitative Analytik ausgehend von der genomischen DNA, die in einer einzigen Zelle enthalten ist. Eine solche Analytik ermöglicht auch andere Verfahren.

Das erfindungsgemäße Verfahren ist nicht darauf beschränkt, lediglich in Verbindung mit dem Material einer Einzelzelle verwendet zu werden. Dies stellt nur eine Anwendung des erfindungsgemäßen Verfahrens da. Das Verfahren ist nämlich grundsätzlich dazu geeignet, überall da zum Einsatz zu kommen, wo es um den Nachweis von Teilmengen eines genetischen Materials und deren relative Häufigkeit innerhalb eines gesamten genetischen Materials geht. Für derartige Nachweise mag es weitere Verfahren geben. Von den bekannten Verfahren hat aber keines die Merkmale und die Vorteile des erfindungsgemäßen Verfahrens und wäre auf eine einzelne Zelle anwendbar.

Die vorstehende Aufgabe, die Merkmale und Vorteile nach der vorliegenden Erfindung können unter Berücksichtigung der folgenden, detaillierten Beschreibung der Figuren, bevorzugter Ausführungsformen und eines Ausführungsbeispiels der vorliegenden Erfindung besser verstanden werden.

Es zeigen:
- Fig. 1: ein Flußdiagramm, das ein Auswahlverfahren für die Auswahl von Zielse- quenzen zum Gegenstand hat,
- Fig. 2: ein Flußdiagramm, das ein Auswahlverfahren für die Auswahl von Primerbin- dungsstellen und Primern zum Gegenstand hat,
- Fig. 3: schematisch die Leuchtintensität einer Auswahl von Messpunkten auf der Oberfläche eines erfindungsgemäßen Mikroarrays,
- Fig. 4: eine Draufsicht auf ein Elektrophoresegel gemäß einem Ausführungsbeispiel der Erfindung,
- Fig. 5: eine Draufsicht auf eine Bande in einem Elektrophoresegel, und
- Fig. 6a, 6b: schematische Darstellungen der Oberflächen erfindungsgemäßer Mikro- arrays.

Hinsichtlich vorstehend im einzelnen nicht näher erläuterter Merkmale der Erfindung wird im übrigen ausdrücklich auf die Patentansprüche und die Figuren verwiesen.

### Genaue Beschreibung der Erfindung

Die Erfindung wird im folgenden anhand einer ersten Ausführungsform und der Figuren näher erläutert.

Im Rahmen der ersten Ausführungsform handelt es sich bei dem genetischen Material um die in einem hapluiden Chromosomensatz vorhandene genomische DNA (Spermium), und bei den abgrenzbaren Teilmengen um sämtliche Chromosomen Chr, die in dem Chromosomensatz vorkommen können. Der Chromosomensatz ist eine humanes Spermium, weshalb die Zahl abgrenzbarer Teilmengen 23 beträgt. Dies entspricht der Zahl der möglicherweise in dem Chromosomensatz vorhandenen Chromosomen Chr1 - Chr23.

Für jede abgrenzbare Teilmenge Chr1, Chr2, ....Chr23 gibt es Zielsequenzen, die lediglich Teil einer oder einer begrenzten Anzahl der abgrenzbaren Teilmengen sind. Das bedeutet, dass diese Zielsequenzen einzigartig für das bzw. die jeweiligen Chromosomen sind, auf dem oder auf denen diese Zielsequenzen vorkommen. Für jedes Chromosom gibt es eine Vielzahl solcher spezifischer Zielsequenzen.

Für das erfindungsgemäße Verfahren werden geeignete Primer in einem Auswahlverfahren in zwei Schritten ermittelt.

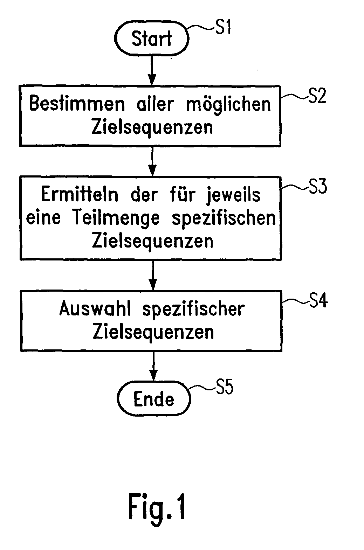

Figur 1 zeigt schematisch ein Auswahlverfahren für die Auswahl von Zielsequenzen. Dieses Verfahren beginnt mit dem Schritt S1. Im Schritt S2 werden alle möglichen Zielsequenzen für alle abgrenzbaren Teilmengen bestimmt. Dies heißt, dass alle möglichen amplifizierbaren Abschnitte in dem genetischen Material ermittelt werden. Danach wird bestimmt (S3), welche dieser Zielsequenzen spezifisch für jeweils eine einzige abgrenzbare Teilmenge sind. Abgrenzbare Teilmengen können z.B. Chromosomen sein. Eine Zielsequenz ist dann spezifisch, wenn sie nur in einer einzelnen, aber nicht mehreren der abgrenzbaren Teilmengen vorkommt.

Für jede abgrenzbare Teilmenge werden mehrere unterschiedliche Zielsequenzen ausgewählt (S4). Vorzugsweise erfolgt die Auswahl der Zielsequenzen nach bestimmten Kriterien. Z.B. werden Zielsequenzen mit hoher Unterscheidungskraft zu den weiteren Zielsequenzen bevorzugt, d.h. Zielsequenzen, die eine möglichst geringe Homologie bzw. Komplementarität zu anderen Zielsequenzen aufweisen, werden bevorzugt ausgewählt. Ferner ist es zweckmäßig, Zielsequenzen auszuwählen, die ähnliche Hybridisierungseigenschaften (z.B. Schmelztemperatur, Bildungsrate) aufweisen.

Mit dem Schritt S5 ist das Auswahlverfahren beendet.

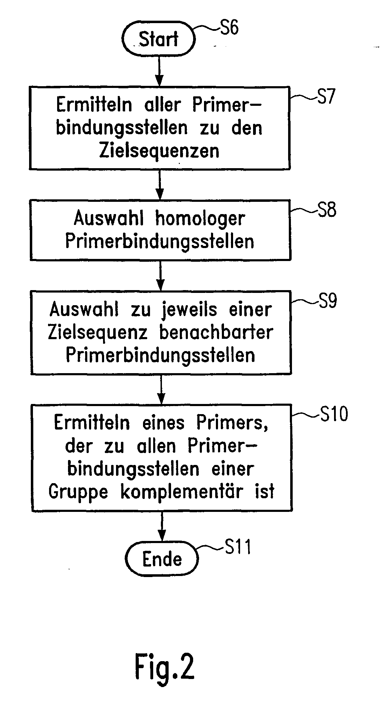

In einem zweiten Verfahrensabschnitt (Fig. 2) wird jeweils ein Primer ermittelt, der zur Amplifikation geeignet ist, und zwar in einem Auswahlverfahren mit folgenden Schritten:
a) Es werden Primerbindungsstellen innerhalb des genetischen Materials ermittelt (S7), die sich in Nachbarschaft zum 3'-Ende der im ersten Schritt ermittelten Zielsequenzen befinden. Bei einer Amplifikationsreaktion wird ein an diese Primerbindungsstellen hybridisierter Primer über die Zielsequenz hinaus verlängert werden und ein Komplement der Zielsequenz erzeugt.
b) Von den in a) ermittelten Primerbindungsstellen werden diejenigen ausgewählt (S8), die im wesentlichen homolog zueinander sind. Dabei werden diejenigen Primerbindungsstellen, die eine geringe Homologie zu anderen der in a) ermittelten Primerbindungsstellen aufweisen, verworfen. Im wesentlichen homolog bedeutet, dass die Primerbindungsstellen eine Homologie von zumindest 80% zueinander aufweisen.
c) Von den in b) ermittelten Primerbindungsstellen werden diejenigen ausgewählt (S9), die sich im wesentlichen nur in Nachbarschaft zum 3'-Ende einer Zielsequenz bzw. deren Komplementes befinden. Im wesentlichen nur bedeutet in diesem Zusammenhang, dass zumindest 50% der Primerbindungsstellen sich in Nachbarschaft zum 3'-Ende einer Zielsequenz bzw. deren Komplementes befinden. Diese Primerbindungsstellen werden zu einer Gruppe von Primerbindungsstellen zusammengefasst. Zu jeder der Primerbindungsstellen dieser Gruppe wird ein Primer ermittelt (S10), der im wesentlichen komplementär zu allen Primerbindungsstellen der Gruppe ist. Im wesentlichen komplementär bedeutet in diesem Zusammenhang, dass der Primer bei entsprechenden Reaktionsbedingungen Hybride mit allen Primerbindungsstellen der Gruppe ausbildet.

Die Primerbindungsstellen der Gruppe umfassen nicht unbedingt die zur Amplifikation aller Zielsequenzen notwendigen Primerbindungsstellen. Hierfür sind an beiden Strängen einer Zielsequenz jeweils eine Primerbindungsstelle in Nachbarschaft zum jeweiligen 3'-Ende der Zielsequenz notwendig, so dass die Zielsequenz von jeweils zwei Primerbindungsstellen flankiert ist.

Falls dies der Fall ist, kann der zweite Verfahrensabschnitt wiederholt werden, um eine oder mehrere weitere Gruppen von Primerbindungsstellen und die dazugehörigen Primer zu ermitteln.

Auf diese Art und Weise werden einer oder einige wenige Primer ausgewählt, die Hybride mit allen zur Amplifikation der Zielsequenzen notwendigen Primerbindungsstellen bilden. Das heisst, dass die Primerbindungsstellen im wesentlichen nur in Nachbarschaft zum 3'-Ende der Zielsequenzen bzw. deren Komplementen befindlich sind.

Im wesentlichen nur Zielmoleküle zu amplifizieren bedeutet, dass zumindest 50% der amplifizierten Moleküle Zielmoleküle sind, die Zielsequenzen umfassen, die spezifisch für zumindest eine abgrenzbare Teilmenge sind, aber nicht auf alle abgrenzbaren Teilmengen zurückgehen können.

Es versteht sich, dass das dargestellte Auswahlverfahren diversen Iterationsprozessen unterworfen werden kann - d.h. dass verschiedene der genannten Kriterien unterschiedlich gewichtet werden können und einzelne Schritte vertauscht oder in Abhängigkeit von davor erhaltenen Ergebnissen mehrfach durchgeführt werden können. Insbesondere kann das auch bedeuten, dass in einem ersten Schritt bekannte unspezifische Primer verwendet werden, die die Amplifikation der oben beschriebenen Zielsequenzen ermöglichen und erst danach geprüft wird, ob sie den Kriterien (Spezifität der Zielsequenzen, Unterscheidungskraft, ähnliche Hybridisierungseigenschaften, usw. ) des ersten Schrittes entsprechen.

Im Rahmen des dargestellten Auswahlverfahrens können auch unspezifische Primer nach dem Stand der Technik, z.B. wie sie im Rahmen der DOP-PCR oder der Inter-ALU-PCR verwendet werden, so modifiziert werden, dass sie den aufgeführten Auswahlkriterien entsprechen.

Das zur Verfügung stehende genetische Material des Chromosomensatzes wird einem Amplifikationsverfahren unterworfen. Dabei bindet der oder binden die Primer in jedem Hybridisierungsschritt an Primerbindungsstellen, die sich in Nachbarschaft zum 3'-Ende von Zielsequenzen befinden, so dass im wesentlichen nur Zielmoleküle amplifiziert werden, die die Zielsequenzen umfassen.

In dem Amplifikationsprodukt wird jedes Chromosom durch eine für das jeweilige Chromosom spezifische Zahl unterschiedlicher Zielsequenzen, die für dieses spezifisch sind, repräsentiert.

Die Amplifikationsreaktion folgt der Formel Y=Sx(1+E)ⁿ, worin Y die Zahl der entstehenden Kopien einer amplifizierten Zielsequenz, E die Effizienz der Amplifikation, n die Anzahl der Zyklen, und S die Zahl der ursprünglich vorhandenen "Startkopien" einer jeweiligen Zielsequenz sind (eine für ein Chromosom spezifische Zielsequenz kann auf dem jeweiligen Chromosom mehrfach vorkommen).

Im Spermium oder in einem Polkörperchen einer normal entwickelten Eizelle liegen die Chromosomen jeweils nur einmal vor. Bei Chromosomenfehlverteilungen liegen bestimmte Chromosomen in einer davon abweichenden Anzahl vor, z.B. 0 oder 2.

Das bedeutet, dass bei Amplifikationen von Zielsequenzen mit nur einem einzelnen Molekül als Startkopie (S=1) für eine quantitative Aussage experimentell gewährleistet werden muss, dass im ersten Zyklus der Amplifikation eine definierte chromosomenspezifische Zielsequenz mit Sicherheit erfasst und amplifiziert wird, was bezügliche eines einzelnen Moleküls aber generell nicht möglich ist. Schlägt der erste Zyklus fehl, so wird am Ende nur die Hälfte der Kopien dieser Zielsequenzen amplifiziert sein. Der Fehler bei der Amplifikation kann in einem größeren Bereich liegen, in dem auch quantifiziert werden soll (Faktor 1,2,3,...), wie häufig ein Chromosom in einem einem Spermium oder Polkörperchen vertreten ist. Quantitative Aussagen bei einem einzigen Molekül als Startsequenz sind dadurch mit so hohen Unsicherheiten behaftet, dass sie eigentlich wertlos sind.

Desgleichen gilt für die Effizienz E, dass sie nur im Idealfall 1 beträgt, d.h. dass in jedem Zyklus der Amplifikation eine Verdopplung des Ausgangsmateriats, also sämtlicher vorhandener Kopien stattfindet. In der Realität herrscht jedoch nie ideale Effizienz, und der Wert für E muss immer < 1 angesetzt werden. Die Effizienz ist im übrigen von einer Vielzahl schwer zu kontrollierender Faktoren abhängig, z.B. von der jeweils amplifizierten Sequenz und der Länge der amplifizierten Bereiche eines Genoms. Sie variiert grundsätzlich von Experiment zu Experiment. Kleine Abweichungen der Effizienz E von der Idealeffizienz einer Amplifikation 1 verursachen sehr große Effekte bei typischen Zyklenzahlen von Amplifikationsverfahren von n=20 - 30.

Mit Hilfe des beschriebenen Verfahrens wird für jedes im Spermium oder im Polkörperchen vorhandene Chromosom eine Vielzahl von unterschiedlichen Zielsequenzen amplifiziert, die fast alle (mindestens ca. 80%) spezifisch für zumindest ein Chromosom sind, und zwar mit Hilfe eines oder weniger Primer. Experimentelle Unwägbarkeiten, die sich aus der von Experiment zu Experiment schwankenden Effizienz von Amplifikationsverfahren ergeben, werden dadurch ausgeschlossen, dass sämtliche Zielsequenzen in einem einzigen Verfahren simultan amplifiziert werden. Fehler der Amplifikation, die sich daraus ergeben, dass bestimmte Zielsequenzen eines Chromosoms im ersten Zyklus nicht amplifiziert werden, werden dadurch ausgeglichen, dass auf jeden Fall ein wesentlicher Teil der Zielsequenzen, die für ein Chromosom spezifisch sind, im ersten Schritt amplifiziert wird.

Wenn z.B. im ersten Chromosom Chr1 eines Chromosomensatzes 26 Zielsequenzen a - z enthalten sind, die nur auf diesem Chromosom vorkommen und die mit Hilfe des beschriebenen Verfahrens mit einem oder wenigen Primern simultan amplifiziert werden, und die Zielsequenzen a, b im ersten Zyklus der Amplifikation nicht amplifiziert werden, dafür aber die Zielsequenzen c - z im ersten Schritt amplifiziert werden, so fällt der Fehler bezüglich der Zielsequenzen a, b im Amplifikationsprodukt nicht ins Gewicht, sofern am Ende die Gesamtheit der amplifizierten, für das Chromosom spezifischen Zielsequenzen a - z herangezogen wird, um eine Aussage über die Quantität des Chromosoms im Spermium oder im Polkörperchen zu treffen.

Das Amplifikationsprodukt kann anschließend mit einem DNA-Chip in Verbindung gebracht werden, auf dessen Oberfläche sich in Reihen und Spalten angeordnete Spots befinden, die jeweils gleiche Fänger-Moleküle aufweisen. Die Fänger-Moleküle können Fänger-Zielsequenz-Hybride mit den jeweiligen Zielsequenzen ausbilden. Dabei ist für jede oder für die überwiegende Zahl der Zielsequenzen ein entsprechender Spot auf dem Chip vorgesehen. Die Spots sind in Abhängigkeit von den darauf befindlichen Sonden-Molekülen spezifisch für eine Zielsequenz und damit spezifisch für zumindest ein Chromosom.

Wenn das Amplifikationsprodukt unter Hybridisierungsbedingungen auf einen solchen DNA-Chip aufgetragen wird, werden Fänger-Zielsequenz-Hybride gebildet. Diese werden anschließend detektiert. Sofern die Amplifikation unter Verwendung von mit Fluoreszenzmarkern versehenen Nukleotidtriphosphaten durchgeführt wurde, bietet sich die Messung der Fluoreszenzintensität der einzelnen Spots an.

Diejenigen Spots CHr1a - Chr1 z, die den Zielsequenzen a - z des Chromosoms Chr1 zuzuordnen sind, werden,
- sofern das Chromosom keinmal im Chromosomensatz vorhanden war, gar keine oder nur eine sehr geringe Fluoreszenz aufweisen, die auf Verunreinigungen zurückzuführen ist,
- sofern das Chromosom ein- oder mehrmals im Chromosomensatz vorhanden war, im Mittel eine Fluoreszenzintensität I_{Chr1} aufweisen.

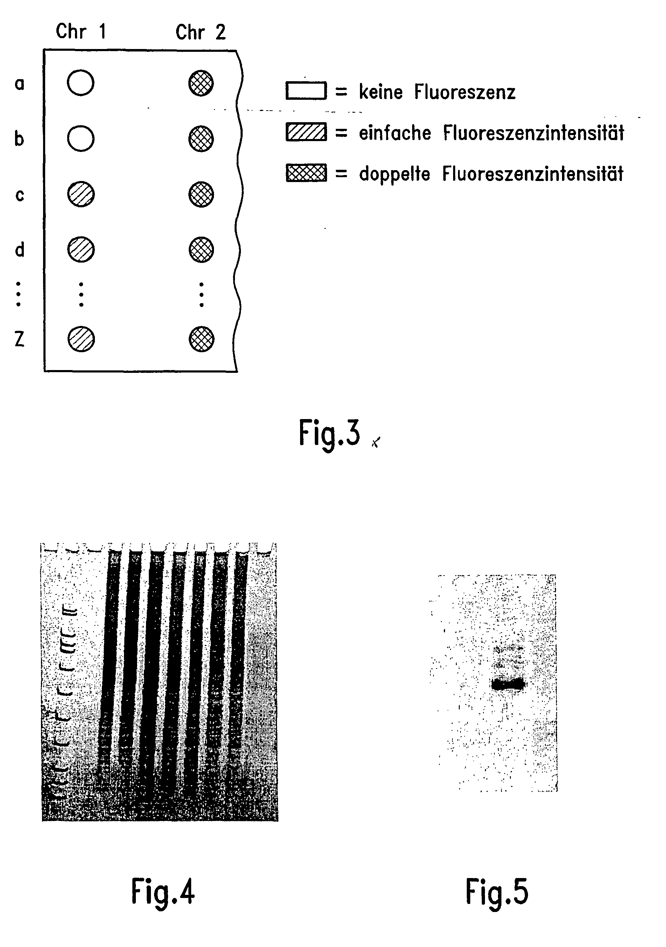

Falls Zielsequenzen a, b des Chromosoms 1 mit schlechter Effizienz amplifiziert werden, führt dies zu Spots Chr1a, Chr1b, in denen keine oder nur eine geringe Fluoreszenzintensität gemessen wird, in Figur 3 dargestellt als Messpunkte ohne Schraffur. Falls die übrigen Zielsequenzen c-z mit hoher Effizienz amplifiziert werden, wird in den Spots Chr1c - Chr1 z eine entsprechend hohe Fluoreszenzintensität gemessen werden, in Figur 3 dargestellt als Messpunkte mit Linienschraffur. Sofern das Chromosom Chr1 in dem Chromosomensatz einmal vorhanden und das Chromosom 2 in dem Chromosomensatz zweimal vorhanden war, wird die mittlere Intensität I_{Chr1} der Fluoreszenz der dem Chromosom 1 zugeordneten Spots Chr1a - Chr1z etwa halb so groß sein wie die mittlere Intensität I_{Chr2} der Fluoreszenz der dem Chromosom 2 zugeordneten Spots Chr2a - Chr2z, in Figur 3 mit Kreuzschraffur dargestellt.

Es kann vorkommen, dass eine Zielsequenz aa, die für das Chromosom Chr1 spezifisch ist, zugleich spezifisch für ein weiteres Chromosom ist, nicht jedoch für alle Chromosomen eines Chromosomensatzes. Wenn beide Chromosomen gleich häufig in einer Probe vorkommen, wird die Intensität der in dem dieser Zielsequenz aa zugeordneten Spot gemessenen Fluoreszenz ungefähr das zweifache dessen betragen, das in Spots gemessen wird, deren Zielsequenz nur auf einem Chromosom vorkommt.

Für die Analyse des Produktes der beschriebenen Amplifikation stehen dem Fachmann eine Vielzahl weiterer Hybridisierungsexperimente zur Verfügung. So können die amplifizierten Sequenzen zum Beispiel auch mittels Elektrophoresemethoden, Kappilarelektrophorese oder Massenspektrometrie analysiert werden.

Nachfolgend wird die Erfindung anhand einer zweiten Ausführungsform näher erläutert.

Die genomische Information eines humanen Chromosomensatz wird mittels eines Amplifikationsverfahrens amplifiziert, wobei das Amplifikationsprodukt eine Vielzahl von Zielsequenzen enthält, und wobei jedem im Polkörperchen vorhandenen Chromosom Zielsequenzen zugeordnet werden können, die nur auf diesem Chromosom vorkommen oder von diesem stammen können.

Das Amplifikationsprodukt wird mit einem DNA-Chip in Verbindung gebracht, auf dem jedes Chromosom durch 10 Spots repräsentiert wird. Dabei sind in einem Spot jeweils Fänger-Oligonukleotide enthalten, die mit Zielsequenzen Hybride ausbilden können, die spezifisch für ein Chromosom sind. Dabei sind in einem Spot 10 unterschiedliche Fänger-Oligonukleotide enthalten, die mit Zielsequenzen Hybride ausbilden können, die von einander unterschiedlich sind, aber alle demselben Chromosom zugeordnet werden. Dasselbe gilt für die weiteren neun Spots, die dem selben Chromosom zugeordnet sind. Bei einem Chromosom Chrn, von dem 26 Zielsequenzen a - z durch Fänger-Oligonukleotide auf dem Chip eingefangen werden können, sind die Spots wie folgt gemischt: im ersten Spot Chrn/1 befinden sich Fängermoleküle für die Zielsequenzen a - j, im zweiten Spot Chrn/2 befinden sich Fänger-Oligonukleotide für die Zielsequenzen j - t, im dritten Spot Chrn/3 befinden sich Fänger-Oligonukleotide für die Zielsequenzen u - d, im vierten Spot CHrn/4 befinden sich Fänger-Oligonukleotide für die Zielsequenzen e - o, im fünften Spot Chrn/5befinden sich Fänger-Oligonukleotide für die Zielsequenzen p - z, im sechsten Spot Chrn/6 befinden sich Fänger-Oligonukleotide für die Zielsequenzen a, c, e, g, i, k, m, o, q, t, im siebten Spot Chrn/7 befinden sich Fänger-Oligo-nukleotide für die Zielsequenzen b, d, f, h, j, l, n, p, r, t, im achten Spot Chrn/8 befinden sich Fänger-Oligonukleotide für die Zielsequenzen m, n, o, p, q, r, w, x, y, z, v, im neunten Spot Chrn/9 befinden sich Fänger-Oligonukleotide für die Zielsequenzen a, e, i, j, m, n, o, p, r, s, und im zehnten Spot Chrn/10 befinden sich Fänger-Oligonukleotide für die Zielsequenzen a, b, c, d, e, v, w, x, y, z. Für jedes der in einem Chromosomensatz möglicherweise vorhandenen 23 Chromosomen Chr1 - Chr23 eines Chromosomensatz es aus einer humanen Eizelle sind auf dem Chip 10 solche Spots Chrn/1 - Chrn/10 vorgesehen, auf denen jeweils 10 von 26 Fänger-Oligonukleotiden wie eben angeführt gemischt sind, die mit Zielsequenzen hybridisieren können, die im Rahmen einer unspezifischen Amplifikation grundsätzlich amplifiziert werden, wenn das Chromosom, für das die jeweiligen Zielsequenzen spezifisch sind, in dem Chromosomensatz vorhanden ist.

Das Amplifikationsprodukt wird auf den DNA-Chip aufgetragen. Dabei hybridisieren die Fänger-Oligonukleotide mit den zu diesen komplementären Zielsequenzen a - z jeweils eines Chromosoms. Im Rahmen der Amplifikation wurde ein Markierungsmittel in die amplifizierten Zielsequenzen eingebaut (ein Cy-3 Fluoreszenzmarker). Der Chip wird gewaschen, und es wird simultan die Fluoreszenz der einzelnen Spots erfasst. Dabei wird die Intensität I_{Chrn/x} jeden einzelnen Spot x, der einem Chromosom n zugeordnet wird, erfasst. Sämtliche Intensitäten I_{Chrn/x} eines Chromosoms n werden zur Mittelwertbildung der Intensität der Spots, die für ein Chromosom spezifisch sind, herangezogen (resultierende mittlere Intensität: Iₙ). Die Intensitäten I₁ - I₂₃ werden mit einander verglichen. Beträgt die Größenordnung der mittleren Intensität der Spots, die einem Chromosom zugeordnet werden, ungefähr = 0, so ist dieses Chromosom nicht im Chromosomensatz enthalten. Beträgt die mittlere Intensität der Spots, die einem Chromosom zugeordnet werden, einen Wert, welcher der Mehrzahl der übrigen Intensitäten entspricht, so wird daraus geschlossen, dass das Chromosom, dem diese Spots zugeordnet sind, in dem Chromosomensatz genau einmal vorkommt. Beträgt die mittlere Intensität der Spots, die ein Chromosom repräsentieren, das doppelte, dreifache oder mehrfache der übrigen Intensitäten, so ist davon auszugehen, dass diese Chromosomen in dem Chromosomensatz zwei-, drei-, viermal oder öfter vorkommen.

Die Häufigkeit bestimmter Zielsequenzen innerhalb eines Chromosoms kann hoch oder niedrig sein. Diese Häufigkeit ist ggf. durch Ermittlung eines entsprechenden Faktors zu berücksichtigen, bzw. der Einfluss der Häufigkeit von Startkopien einer Zielsequenz auf die Bildung bestimmter Hybride in einem Spot nach Durchführung einer parallelen Amplifikation. Die Häufigkeit der Zielsequenzen eines bestimmten Chromosoms kann auch von der Größe des jeweiligen Chromosoms abhängen. Daraus resultierende Effekte sind ggf. ebenfalls in einen entsprechenden Korrekturfaktor einzubeziehen, der bei der Auswertung herangezogen wird.

Das alle Chromosomen eines Chromosomensatzes in diesem zweimal vorkommen, ist sehr unwahrscheinlich, weshalb die anhand eines erfindungsgemäßen Verfahrens getroffene Aussage über die Quantität der Chromosomen in einem Chromosomensatz recht sicher ist. Um diese Sicherheit zu erhöhen, kann aber parallel eine Referenzprobe ko- oder parallel amplifiziert und simultan mit der zu untersuchenden Probe analysiert werden.

Fällt einer der Spots eines solchen DNA-Chips aus Produktionsgründen aus, z.B. weil er schlecht gespottet wurde, so stehen noch neun weitere Spots zur Verfügung, um Aussagen über die relative Quantität eines Chromosoms in dem Chromosomensatz zu treffen. Durch die Mischung verschiedener für ein Chromosom, aber für unterschiedliche Zielsequenzen hieraus spezifische Fänger-Sequenzen in einem Spot wird - auch bei ungleichen Effizienzen der Amplifikation bezüglich der jeweiligen Zielsequenz - jeder Spot eine messbare Intensität aufweisen - sofern entsprechendes Ausgangsmaterial im Chromosomensatz vorhanden war - die einem statistischen Mittelwert entspricht. Jeder Spot für sich genommen ist daher schon aussagekräftiger als ein Spot, in dem lediglich eine Art von Fänger-Molekülen vorgesehen wäre. Durch das Vorsehen mehrerer derartiger gemischter Spots pro Chromosom, die ausserdem unterschiedlich gemischte Fänger-Moleküle aufweisen, lassen sich Ungenauigkeiten bei der Amplifikation besser ausschließen als nach bisherigen Verfahren.

Setzt man die gemessenen Intensitäten der ersten, zweiten, dritten .... zehnten gemischten Spots 1, 2, 3, ... 10 die jeweils einem der Chromosomen Chr1 - Chr23 eines Chromosomensatzes zugeordnet werden, zueinander in Relation, so erhält man 10 verschiedene Aussagen über das quantitative Vorkommen der bis zu 23 in einem humanen Chromosomensatzes normalerweise vorkommenden Chromosomen. Das entspricht einer mehrfachen Verifikation eines Analyseergebnisses.

Anstelle von 10 unterschiedlichen Spots wie eben dargestellt kann für jedes Chromosom auch lediglich ein Spot vorgesehen sein, der - nach einer Variante des Ausführungsbeispiels - 26 Fänger-Moleküle enthält, die den Zielsequenzen a-z eines Chromosoms entsprechen. Eine rechnerische Mittelwertbildung erübrigt sich - der Mittelwert der Intensität von allen für ein Chromosom spezifischen Hybriden kommt durch die in einem Spot jeweils vorgesehene Mischung der Fänger-Moleküle zustande.

Aus der ermittelten Anzahl der in einem Chromosomensatz vorhandenen Chromosomen lässt sich direkt auf die Anzahl an Chromosomen in der Eizelle schließen. Auf diese Weise kann die chromosomale Integrität einer Eizelle mit hoher Sicherheit festgestellt werden.

Fängermoleküle im Rahmen der Erfindung bestehen bevorzugt aus synthetischen Oligonukleotiden. Sie können aber auch umfassen: DNA, cDNA, RNA, aRNA, LNA, und/oder anderweitig modifizierte Nukleinsäuren.

### Ausführungsbeispiel

Im folgenden wird die Erfindung anhand eines konkreten Ausführungsbeispiels beschrieben. Für die Amplifikation des Chromosenmaterials, das jeweils aus einer Einzelzelle isoliert wurde, wurde folgender Primer gemäß dem Verfahren aus Figur 2 ausgewählt:
Ale1-k 5'-CCAAAGTGCTGGGATTACAG-3'

Mit dieser Primersequenz wird eine PCR-Amplifikation unter folgenden Bedingungen durchgeführt: mehrere unterschiedliche Proben werden zunächst fünf Minuten auf 95°C erwärmt, anschließend 35 mal 30 Sekunden auf 95°C, 30 Sekunden auf 62°C und 30 Sekunden auf 72°C erwärmt. Am Ende des letzten Zyklus werden die Proben 10 Minuten auf 72°C temperiert und anschließend auf 4°C abgekühlt.

Der Primer Ale1-k hat sich als außerordentlich effizient bei der Durchführung des erfindungsgemäßen Verfahrens erwiesen. Beim Tausch lediglich einer Base gegen eine andere Base mag der Primer noch seine Funktion erfüllen, insbesondere wenn lediglich die endständigen Primerbereiche betroffen sind. Auch das Weglassen zweier endständiger Basen am Primer mag noch zu brauchbaren Ergebnissen führen. Derartige Abwandlungen, die dem Fachmann bekannt sind, führen aber zu erheblichen Qualitätsverlusten. Wenn mehr als zwei Basen des erfindungsgemäßen Primers ausgetauscht oder weggelassen werden, ist das erfindungsgemäße Verfahren kaum mehr durchführbar. Dass der Primer seine Funktion erfüllt, wird im folgenden anhand der Figuren 4 und 5 erläutert.

Die Amplifikationsprodukte werden auf ein Gel aufgetragen und einer Gelelektrophorese unterworfen. Eine Aufsicht auf das resultierende Elektrophoresegel ist in Figur 4 wiedergegeben. Darauf sind von links nach rechts angeordnet 9 Spuren 1 - 9 zu erkennen. Spur 1 ist der Molekulargewichtsstandard, Spur 2 eine Negativkontrolle, und die Spuren 3 - 9 identische Muster unterschiedlicher Proben mit jeweils einer hapluiden Zelle als Ausgangsmaterial.

Auf dem in Figur 4 gezeigten Gel sind Sequenzen nachweisbar, die in silico gemäß einem Verfahren wie in Figur 1 dargestellt vorhergesagt wurden.

Beispielhaft werden zwei Sequenzen genannt: SHGC-6833 und RH102636, die unter ihrer jeweiligen Bezeichnung in der NCBI Datenbank zu finden sind. Beide Sequenzen sind Teil der mittels Ale1-k amplifizierten spezifischen Sequenzen. SHGC-6833 ist spezifisch auf Chromosom 21 zu finden. Die Sequenz (im folgenden als sequence tagged site sequence bzw. STS sequence bezeichnet) von SHGC-6833 lautet:
acagaaaggtggaggaaaagttagagcaatattttttggtttatagctggctttggggaaaacggattctggtttc
tatgcctagcctcagggaaacgtgagatggataacatgagggcaggagaaggtcagacga
aaacttttgcttccaaggtctttgttttgagtatcattttctgaatcccgacattccctg
gtctgaaactttcccaagaagtttcacagtccagaaattggattggt

Hybridisiert man mit einer markierten STS-Sonde, das heißt einem Komplement zur STS-Sequenz, in das ein Markierungsmittel eingebaut ist, auf das in Figur 4 gezeigte Gel (Spur3), so erhält man ein spezifisches Signal der erwarteten Grösse, wie in Figur 5 dargestellt. Dieses Signal ist der Nachweis für das Vorhandensein von SHGC-6833 im Ausgangsmaterial und damit für das Vorhandensein von Chromosom 21.

RH102636 ist spezifisch auf Chromosom 1 zu finden. Die Sequenz von RH-102636 lautet wie folgt:
ccatgtaacacaagctcacagcctctaatgttaccaaccttataca
caaatggccaaacaagaaattgtcctttccaaaagataatttattctggtttcccctcttca

Der Nachweis von RH-102636 auf dem Gel ist zugleich der Nachweis dafür, dass RH-102636 im Ausgangsmaterial vorhanden war und damit der Nachweis für das Vorhandensein von Chromosom 1.

Die jeweilige Sequenz kommt nur ein einziges mal auf dem jeweiligen Chromosom vor. Dort, wo die Fluoreszenzintensität der beiden Sequenznachweise etwa gleich ist, lässt sich die Aussage treffen, dass die Chromosomen 1 und 21 in der betroffenen Probe zu gleichen Teilen vorhanden sind.

In silico wurden weitere Sequenzen vorhergesagt, von denen jede nur ein einziges Mal auf einem bestimmten Chromosom vorkommt. Eine Übersicht über die bisher in silico vorhergesagten Sequenzen ergibt sich aus Tabelle 1.

**Tabelle 1**

| Zusammenstellung aller in silico vorhergesagten Sequenzen | | | |
|---|---|---|---|
| Chromosom | PCR-Produkte | davon STS-Sequenzen | Zahl der Primerbindungsstellen |
| Chr.1 | 4512 | 30 | 71485 |
| Chr.2 | 3016 | 8 | 59619 |
| Chr.3 | 2245 | 10 | 45920 |
| Chr.4 | 1664 | 11 | 38816 |
| Chr.5 | 2076 | 16 | 40794 |
| Chr.6 | 2124 | 6 | 41695 |
| Chr.7 | 3350 | 31 | 49076 |
| Chr.8 | 1608 | 9 | 34000 |
| Chr.9 | 1966 | 8 | 33818 |
| Chr.10 | 2268 | 9 | 39827 |
| Chr.11 | 1894 | 11 | 34259 |
| Chr.12 | 2429 | 17 | 40262 |
| Chr.13 | 1195 | 7 | 26921 |
| Chr.14 | 2162 | 22 | 34280 |
| Chr.15 | 2198 | 12 | 35623 |
| Chr.16 | 3677 | 8 | 46664 |
| Chr.17 | 4255 | 23 | 52336 |
| Chr.18 | 984 | 4 | 21969 |
| Chr.19 | 6049 | 24 | 54283 |
| Chr.20 | 1958 | 10 | 27451 |
| Chr.21 | 571 | 15 | 11533 |
| Chr.22 | 1872 | 9 | 22584 |
| Chr.X | 1834 | 8 | 32452 |
| Chr.Y | 160 | 1 | 4585 |

Darin ist in der ersten Spalte das jeweilige humane Chromosom bezeichnet. In der zweiten Spalte sind zu den jeweiligen Chromosomen die Anzahl der unterschiedlichen in silico vorhergesagten Amplifikationsprodukte einer Amplifikation mit Ale1-k genannt. Fast alle der in der zweiten Spalte enthaltenen Amplifikationsprodukte sind spezifisch. In der dritten Spalte ist die Zahl der bisher bekannten und veröffentlichten, für das jeweilige Chromosom spezifischen Sequenzen (STS-Sequenzen) aufgeführt, die in öffentlichen Datenbanken zugänglich sind, und die jeweils eine Teilmenge der jeweiligen PCR-Produkte in Spalte 2 darstellen. In der vierten Spalte ist die Zahl der Primerbindungsstellen für Ale1-k auf dem jeweiligen Chromosom angeführt. Da der Primer nicht immer eine Bindungsstelle in einer für eine erfolgreiche Amplifikation eines Abschnitts erforderlichen Nähe zu einer ersten Bindungsstelle hat, an der er auch komplementär in umgekehrter Richtung hybridisieren kann, entsteht nicht immer automatisch ein PCR Produkt. Beim erfindungsgemäßen Primer Ale1-k ist dies nur bei einem Bruchteil der Fall. So könnte man annehmen, dass bei 71485 Primerbindungsstellen des Chromosom 1 etwa 35000 PCR-Produkte entstünden, deren Anzahl aber lediglich 4512 beträgt.

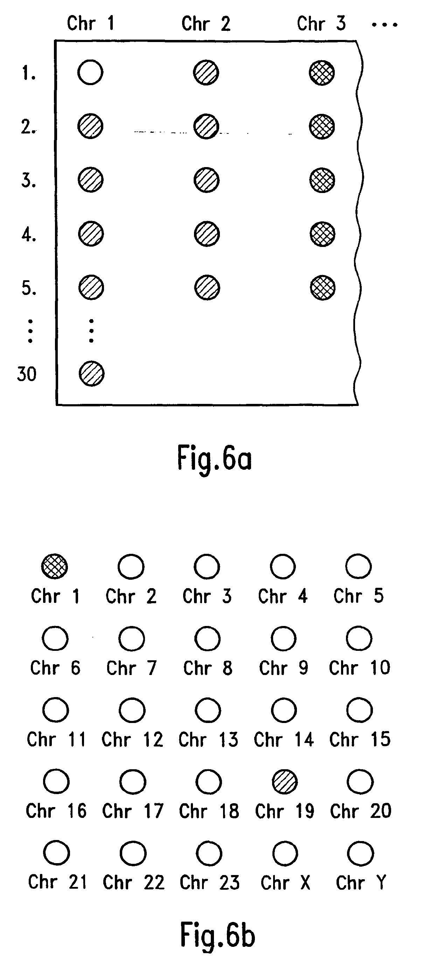

Ein DNA-Chip oder Mikroarray, der zur Analyse eines aus einer erfindungsgemäßen Amplifikation erhaltenen Reaktionsgemisches eingesetzt wird, kann ausgestaltet sein wie in Figur 6a oder Figur 6b schematisch dargestellt.

Eine Möglichkeit besteht darin, für jede STS-Sequenz lediglich einen separaten Messpunkt vorzusehen. In einem solchen Messpunkt befinden sich nur Fängermoleküle, die spezifisch mit der entsprechenden STS-Sequenz oder einem Abschnitt davon ein Hybrid eingehen. Ein Fluoreszenznachweis in einem Messpunkt bedeutet dann, dass diese Sequenz in der Probe vorhanden war. Für das Chromosom 1 können beispielsweise bis zu 30 unterschiedliche Messpunkte auf einem Mikroarray vorgesehen sein. Wenn bei der Amplifikation eine von 30 der auf dem Mikroarray für das Chromosom 1 nachweisbaren STS-Sequenzen schlecht amplifiziert wird, beispielsweise, weil im ersten Amplifikationszyklus bei dieser Sequenz der Primer nicht an die vorgesehene Primerbindungsstelle gebunden hat, stehen noch 29 weitere Sequenzen zur Verfügung, deren Nachweis zugleich ein Nachweis für das Vorhandensein des Chromosoms 1 in der Probe darstellt. Wenn ein derartiger Amplifikationsfehler zu einer Erniedrigung der amplifizierten Menge dieser Sequenz in Bezug auf die anderen Sequenzen führt, wird in dem Messpunkt, der diese Sequenz repräsentiert, eine niedrigere Fluoreszenzintensität zu beobachten sein als in den anderen Messpunkten (Messpunkt ohne Schraffur links oben in Figur 6a). Dadurch, dass für 29 weitere, für das Chromosom 1 spezifische Amplifikationsprodukte jeweils ein Messpunkt auf dem Mikroarray vorgesehen ist, kann das fehlerhafte Amplifikationsprodukt als solches identifiziert werden. In die Auswertung für die Bestimmung der relativen Menge des Chromosoms 1 gehen dann lediglich einige der übrigen 29 Messpunkte ein (linienschraffierte Messpunkte 2 - 29 in der ersten Spalte des in Figur 6a gezeigten Mikroarrays). Ist deren Intensität etwa gleichzusetzen mit der Intensität, die in Messpunkten gemessen wird, die spezifisch jeweils eine STS-Sequenz des Chromosoms 2 repräsentieren, so folgt daraus, dass die Mengen der jeweiligen amplifizierten STS-Sequenzen der Chromosomen 1 und 2 in etwa gleich sind (Spalte 2 der Messpunkte in Figur 6a mit Linienschraffur). Daraus folgt wiederum, dass die Chromosomen 1 und 2 in der betroffenen Probe im gleichen Mengenverhältnis zueinander vorliegen. Ist das Chromosom 3 im Vergleich zu den übrigen Chromosomen doppelt so oft in der Probe enthalten, wird in den entsprechenden Messpunkten eine im Vergleich zu den übrigen Messpunkten verdoppelte Fluoreszenzintensität zu beobachten sein (Messpunkte mit Kreuzschraffur, dritte Spalte in Figur 6a). Einzelne Messpunkte, die wegen Amplifikationsfehlern, die bei einer Amplifikation aus einer sehr geringen Menge von Ausgangsmaterial regelmässig vorkommen, keine oder nur geringe Fluoreszenzintensität aufweisen, wie eben bezüglich des ersten Messpunktes in Bezug auf Chromosom 1 dargelegt, sind kein Hindernis für die Analyse, solange zumindest eine STS-Sequenz je Chromosom richtig amplifiziert wird. Die Wahrscheinlichkeit, dass aufgrund von Amplifikationsfehlern alle STS-Sequenzen eines Chromosoms schlechter amplifiziert werden, als die STS-Sequenzen eines anderen Chromosoms, ist statistisch sehr gering. Ein Mikroarray, auf dem in unterschiedlichen Messpunkten jeweils Fängermoleküle vorgesehen sind, die dort in gleichen Konzentrationen vorliegen, und die jeweils Hybride mit einer spezifischen STS-Sequenz aus Tabelle 1 ausbilden, ist darum hervorragend dazu geeignet, Aussagen über die relative Menge der Chromosomen in einer Probe, die mittels Ale1-k amplifiziert wurde, auf schnelle und zuverlässige Weise zu treffen.

In einem Messpunkt eines erfindungsgemäßen Mikroarrays können auch Fängermoleküle enthalten sein, die mit allen STS-Sequenzen, die für ein bestimmtes Chromosom spezifisch sind, oder mit eine bestimmten Anzahl derartiger Sequenzen Hybride eingehen können. Ein solcher Mikroarray ist schematisch in Figur 6a dargestellt. Zu jedem Spot ist angegeben, zum Nachweis welchen Chromosoms er dienen soll. Wenn in einem einzelnen Messpunkt alle STS-Sequenzen nachweisbar sind, die jeweils für eines der Chromosomen aus Tabelle 1 spezifisch sind, verhalten sich die relativen Intensitäten der nachgewiesenen Hybride bei der Signal-Auswertung wie die Zahl von je Chromosom nachgewiesenen STS-Sequenzen zueinander (also maximal etwa 1:10, Chromosom 19 : Chromosom 1, in Figur 6b dargestellt durch eine Kreuzschraffur im Spot Chr1 und eine Linienschraffur im Spot Chr19; die übrigen Spots oder Messpunkte sind aus Gründen der Übersichtlichkeit nicht schraffiert).

Geeignet ist auch ein Mikroarray, in dem in einzelnen Messpunkten unterschiedliche, aber nicht alle STS-Sequenzen, die für ein Chromosom spezifisch sind, nachweisbar sind. Bei der Auswertung der gemessenen Intensitäten sind diese entsprechend zu gewichten. Schließlich können auf einem Mikroarray verschiedene Arten von Messpunkten integriert sein, d.h. der Mikroarray kann Messpunkte aufweisen, die denen in Figur 6a entsprechen, Messpunkte, die denen in Figur 6b entsprechen, oder Messpunkte, wie eben beschrieben. Durch die Integration einer Vielzahl unterschiedlich ausgestalteter Messpunkte auf einem einzigen Mikroarray stehen alle möglichen der vorgestellten Arten der Auswertung zur Verfügung, so dass die Ergebnisse sich besser verifizieren lassen. Hierdurch werden erfindungsgemäße Verfahren besonders zuverlässig.

In den angeführten Ausführungsformen und im Ausführungsbeispiel wurde die Erfindung am Beispiel einer Spermiumanalyse erläutert. Beschriebene Verfahren können auch auf anderes genetisches Material als das in einem Spermium enthaltene Genom, insbesondere auf in einer humanen Einzelzelle oder in einem humanen Polkörperchen enthaltene Genom und dessen Chromosomen angewandt werden. Ferner kann das beschriebene Verfahren auf bestimmte Deletionen oder Insertionen als abgrenzbare Teilmengen innerhalb eines genetischen Materials, z.B. innerhalb eines einzelnen Chromosoms oder eines Abschnitts hiervon als genetischem Material, angewandt werden.

### Literatur:

Angell, R.R., Man, J. & Keith, J. 1993: Chromosome anomalies in human oocytes in relation to age. Hum Reprod. 8(7): 1047-54.
Beier, M. & Hoheisel, J. (1999): Nucl. Acids Res. 27: 1970-1977.
Eckel, H., Kleinstein, J., Wieacker, P., Stumm, M (2001): Die zytogenetische Analyse von nicht fertilisierten Oozyten - Möglichkeiten und Grenzen. medizinische Genetik 13: 25-30.
Garvin, AM, Holzgreve, W. & Hahn, S. (1998): Highly accurate analysis of heterozygous laci by single cell PCR. Nucl. Acids Res. 26: 3468-3472.
Griffin, D. K. (1996): The incidence, origin, and etiology of aneuploidy. Int Rev Cytol. 167: 26396.
Grothues, D., Cantor, C.R. & Smith, C. (1993): PCR amplification of megabase DNA with tagged random primers (T-PCR). Nucl. Acids Res. 21: 1321-1322.
Hagen-Mann, K. & Mann, W. (1990): Polymerase Chain Reaction - Eine revolutionäre Methode für die Biologie. BIUZ 20: 257-262.
Hagen-Mann, K. & Mann, W. (1995): RT-PCR and alternative methods to PCR for in vitro amplification of nucleic acids. Exp. Clin. Endocrinol. 103: 150-155.
Hardt, T., H. Himmelbauer, W. Mann, H.M. Ropers & Haaf, T. (1999): Towards identification of individual homologous chromosomes: comparative genomic hybridization and spectral karyotyping discriminating between paternal and maternal euchromatin in Mus musculus x M. spretus interspecific hybrids. Cytogen. Cell Genet. 86:187-193.
Heller, A., Chudoba, L, Bleck, C., SENGER; G., Claussen, U., & Liehr, T. (2000): -CGH of Microdissection based comparative genomic hybridization analysis micro secondary acute myiegenous leukemias. Int. J. Oncol. 16: 461-468.
Huang, Q, Schantz, S.P., Rao, P.H., Mo, J., McCormick, S.A. & Chaganti, R.S. (2000): Improving degenerate oligonucleotide primed PCR-comparative genomic hybridization for analysis of DNA copy number changes in tumors. Genes Chromosomes Cancer 28: 395-403.
Kingsley, K, Wirth, J., van der Maarel, S., Freier, S., Ropers, R-R & Haaf, T. (1997): Complex FISH probes for the subtelomeric regions of all human chromosomes: comarative genomic hybridization of CEPH YACs to chromosomes of the old world monkey Presbytis cristata and great apes. Cytogenet Cell Genet 78: 12-19.
Klein, C.A., Schmidt-Kitler, 0., Schardt, J.A., Pantel, K, Speicher, M.R. & Riethm51ler, G. (1999): Comparative genomic hybridization, loss of heterozygosity, and DNA sequence analysis of single cells. PNAS 96: 4494-4499.
Pollack, JR, Perou, C.M., Alizadeh, A.A., Eisen, MR, Pergamenschikov, A., Williams, C.F., Jeffrey, S.S., Botstein, D. & P.O. Brown (1999): Genome wide analysis of DNA copy number changes using cDNA microarrays. Nature Genetics 23: 41-46.
Schnell, S. & Mendoza, C. (1997): Theoretical description of the polymerase chain reaction. J. theor. Biol. 188: 313-318.
Stolovitzky G. & Cecchi G. (1996): Efficiency of DNA replication in the polymerase chain reaction. PNAS 93:12947-12952.
Swaminathan, N., McMaster, K, Skowron, P.M., & Mead, D. (1998): Thermal cycle labeling: Zeptomole detection sensitivity and microgram probe amplific ation using CvUI restriction generated oligonucleotides. Anal. Biochem. 255: 133-141.
Telenius, H. Carter, N.P., Bebb, C.E., Nordenskj61d, M., Ponder, B.A.J., & Tunnacliff, A. (1992): Degenerate oligonucleotide primed PCR: general amplification of target DNA by a single degenerated primer. Genomics 13: 718-725.
Verlinsky, Y., Cieslak, J., Ivakhnenko, V., Evsikov, S., Wolf, G., White, M., Lifchez, k Kaplan, B., Moise, J., Valle, 1, Ginsberg, N., Strom, C. & Kuliev, A. (1998): Preimplantation diagnosis of common aneuploidies by the first- and second-polar body FISH analysis. J Assist Reprod Genet.: 15(5):285-9.
Voullaire, L., Wilton, L., Slater, H. & Williamson, R. (1999): Detection of aneuploidy in single cells using comparative genomic hybridization. Prenat. Diagn. 19: 846-851.
Wodicka, L., Dong, H, Mittmann, M., Ho, M.-H., Lockhart, D. 1 (1997) Genome-wide expression monitoring in Saccharpmyces cerevisiae. Nature Biotechnol 15: 13591367.
Zenzes, M.T., Wang, P. & Casper, R.F. (1992): Evidence for maternal predisposition to chromosome aneuploidy in multiple oocytes of some in vitro fertilization patients. Fertil Steril. 57(I):143-9.
Zhang, L., Cui, X., Schmitt, K, Hubert, R., Navidi, W. & Arnheim, N. (1992): Whole genome amplification from a single cell: implications for'genetic analysis. PNAS 89: 4847-5851.
Zhou, Y, Wang, H., Wie, J., Cui, L. Deng, X., Wang, X. & Chen, Z. (2000): Comparison of two PCR techniques used in amplification of microdissected plant chromosomes from rice and wheat. Biotechniques 28: 766-774.
Datenbank PubMed bei NCBI, Adresse www.ncbi.nlm.nih.gov, Zusammenfassung zu: Rapid detection of common autosomal aneuploidies by quantitative fluorescent PCR on uncultured amniocytes. RAHIL, H. u.a., Eur. J. Hum. Genet. (August 2002) 10 (8) 462-6.
Datenbank PubMed bei NCBI, Adresse www.ncbi.nlm.nih.gov, Zusammenfassung zu: Identification of chromosomal translocations in leukemias by hybridization with oligonucleotide microarrays. NASEDKINA, T. u.a., Haematologica (April 2002) 87 (4) 363-72.
Datenbank PubMed bei NCBI, Adresse www.ncbi.nlm.nih.gov, Zusammenfassung zu: DNA microarray technology for neonatal screening. DOBROWOLSKI, S.F. u.a., Acta Paediatr. Suppl. (1999) 88 (432) 61-4.
WO 02/44411
WO 00/24925
EP 1 026 260 A1
DE 101 02 687 A1
DE 100 59 776 A1
Abstract zu "Preimplantation Genetic Diagnosis, Polar Body Biopsy" von The First World Congress On: Controversies in Ostetrics, Gynecology & Infertility, Prague, Czech Republic - 1999 von Y. Verlinsky, A. Kuliew.
Journal für Fertilität und Reproduktion, Nummer 4/2002, Seite 7ff, M. Montag, K. van der Ven, H. van der Ven "Erste klinische Erfahrungen mit der Polkörperchendiagnostik in Deutschland".
"Einführung in die Präimplantationsdiagnostik", E. Schwinger, Lübeck, Quelle: http://www.studgen.uni-mainz.de/manuskripte/schwinger.pdf.
Flyer zu Polkörperchendiagnostik der Pränatal-Medizin München, Dr. med Karl-Philip Gloning et al.

## Patentansprüche

1. Verfahren zur Bestimmung der relativen Häufigkeit abgrenzbarer Teilmengen eines genetischen Materials, wobei das genetische Material mittels Polymerase-Ketten-Reaktion amplifiziert wird, bei dem Primer verwendet werden, die komplementär zu Primerbindungsstellen sind, die in dem genetischen Material an mehreren Stellen vorhanden sind, und die jeweils benachbart zu einer für jeweils eine Teilmenge spezifischen Zielsequenz mit vorbestimmter Länge sind, so dass ein Amplifikationsprodukt erhalten wird, das im wesentlichen nur amplifizierte Sequenzen aufweist, die eine für die jeweilige genetische Information spezifische Zielsequenz mit vorbestimmter Länge umfassen, die zur Detektion mittels Hybridisierung geeignet ist, wobei das Amplifikationsprodukt mittels eines Hybridisierungsexperimentes zur Bestimmung der relativen Häufigkeit der abgrenzbaren Teilmengen analysiert wird.

2. Verfahren nach Anspruch 1,
**dadurch gekennzeichnet, dass** die Homologie zwischen den Primern und den jeweiligen Primerbindungsstellen in einem Bereich von 80%-100%, und vorzugsweise in einem Bereich von 90%-100% liegt.

3. Verfahren nach einem der Ansprüche 1-2,
**dadurch gekennzeichnet, dass** im Verlauf der Polymerase-Ketten-Reaktion mit Markierungen versehene Nukleotidbausteine verwendet werden.

4. Verfahren nach einem der Ansprüche 1-3,
**gekennzeichnet durch**
- ein Vermengen des Amplifikationsprodukts mit Fänger-Molekülen, so dass sich Hybride aus Fänger-Molekülen und den spezifischen Zielsequenzen ausbilden, und
- Detektion der Hybride.

5. Verfahren nach Anspruch 4,
**dadurch gekennzeichnet, dass** die Fänger-Moleküle auf einem DNA-Chip angeordnet sind.

6. Verfahren nach Anspruch 4,
**dadurch gekennzeichnet, dass** die Fänger-Moleküle aus Oligonukleotiden ausgebildet sind.

7. Verfahren nach einem der Ansprüche 1-6,
**dadurch gekennzeichnet, dass** darin ein DNA-Chip verwendet wird, bei dem in einem einzelnen Spot jeweils gleiche Fänger-Moleküle vorgesehen sind.

8. Verfahren nach Anspruch 1-7,
**dadurch gekennzeichnet, dass** darin ein DNA-Chip verwendet wird, bei dem in einem einzelnen Spot unterschiedliche spezifische Fänger-Moleküle für unterschiedliche Zielsequenzen vorgesehen sind, die alle einer der abgrenzbaren Teilmengen des genetischen Materials zugeordnet sind.

9. Verfahren zur Bestimmung der relativen Häufigkeit abgrenzbarer Teilmengen eines genetischen Materials nach einem der Ansprüche 1 bis 8, mit den Schritten:
- Durchführen eines Verfahrens zur Amplifikation genetischer Informationen aus genetischem Material, das mehrere voneinander abgrenzbare Teilmengen genetischen Materials umfasst, so dass ein Amplifikationsprodukt erhalten wird, das Zielsequenzen umfasst, die zur Detektion mittels Hybridisierung geeignet sind und den abgrenzbaren Teilmengen zugeordnet werden können,
- Vermengen des Amplifikationsprodukts mit Fänger-Molekülen auf einem DNA-Chip, so dass sich Hybride aus Fänger-Molekülen und Zielsequenzen aus dem Amplifikationsprodukt ausbilden, wobei der DNA-Chip zumindest zwei Gruppen von Spots umfasst, wobei die Spots innerhalb einer Gruppe unterschiedliche Fänger-Moleküle aufweisen und eine Gruppe von Spots jeweils einer der abgrenzbaren Teilmengen des genetischen Materials zugeordnet ist,
- quantitative Detektion der jeweils in einem Spot mit unterschiedlichen Fänger-Molekülen des DNA-Chips gebildeten Hybride, so dass für jeden Spot jeweils ein Detektionswert erhalten wird,
- Mittelwertbildung der Detektionswerte der auf dem DNA-Chip vorhandenen Gruppen von Spots,
- Bestimmung der relativen Häufigkeit von Teilmengen genetischen Materials innerhalb eines genetischen Materials durch Vergleich der Mittelwerte.

10. Verfahren nach einem Ansprüche 1-9,
**dadurch gekennzeichnet, dass** das genetische Material aus einer Einzelzelle stammt oder auf diese zurückzuführen ist.

11. Verfahren nach einem Ansprüche 1-9,
**dadurch gekennzeichnet, dass** das genetische Material ein Chromosomensatz aus einem Polkörperchen einer Eizelle ist.

12. Verfahren nach einem Ansprüche 1-9,
**dadurch gekennzeichnet, dass** eine abgrenzbare Teilmenge aus einem oder mehreren Chromosomen besteht.

13. Verfahren nach einem Ansprüche 1-9,
**dadurch gekennzeichnet, dass** eine abgrenzbare Teilmengen aus einem oder mehreren Genen besteht.

14. Verfahren nach einem der Ansprüche 1-9,
**dadurch gekennzeichnet, dass** genetische Informationen eines genetischen Referenzmaterials unter ansonsten gleichen Reaktionsbedingungen parallel amplifiziert werden, so dass ein Amplifikationsprodukt erhalten wird, das im wesentlichen nur amplifizierte Sequenzen aufweist, die für die jeweilige genetische Information spezifische Zielsequenzen mit vorbestimmter Länge umfassen, die zur Detektion mittels Hybridisierung geeignet sind.

15. Verfahren gemäß einem der Ansprüche 1 - 14, in dem zur Amplifikation der Primer Ale1-k mit der Basenalsfolge 5'-CCAAAGTGCTG GGATTACAG-3'.

## Claims

1. A method for determining the relative frequency of delimitable partial amounts of a genetic material, wherein the genetic material is amplified by means of polymerase chain reaction, wherein primers are used which are complementary to primer binding sites which are present at multiple sites in the genetic material and which are each adjacent to a target sequence of predetermined length specific to a respective partial amount so that an amplification product is obtained which substantially only has amplified sequences which include a target sequence which is of predetermined length and specific to the respective genetic information and which is suitable for detection by means of hybridization, with the amplification product being analyzed by means of a hybridization experiment for the determination of the relative frequency of the delimitable partial amounts.

2. A method in accordance with claim 1, **characterized in that** the homology between the primers and the respective primer binding sites is in a range from 80-100%, and preferably in a range from 90-100%

3. A method in accordance with one of the claims 1 - 2, **characterized in that** nucleotide components provided with markings are used in the course of the polymerase chain reaction,

4. A method in accordance with any one of the claims 1 - 3, **characterized by**
- admixing the amplification product with catcher molecules so that hybrids of catcher molecules and the specific target sequences are formed; and
- detecting the hybrids.

5. A method in accordance with claim 4, **characterized in that** the catcher molecules are arranged on a DNA chip.

6. A method in accordance with claim 4, **characterized in that** the catcher molecules are formed from oligonucleotides.

7. A method in accordance with any one of the claims 1 - 6, **characterized in that** a DNA chip is used therein in which catcher molecules which are respectively the same are provided in an individual spot.

8. A method in accordance with any one of the claims 1 - 7, **characterized in that** a DNA chip is used therein in which different specific catcher molecules are provided in an individual spot for different target sequences which are all associated with one of the delimitable partial amounts of the genetic material.

9. A method for determining the relative frequency of delimitable partial amounts of genetic material in accordance with any one of the claims 1 to 8, comprising the steps:
- carrying out a method for the amplification of the genetic information from genetic material which includes a plurality of partial amounts of genetic material delimitable from one another so that an amplification product is obtained which includes target sequences which are suitable for detection by means of hybridization and can be associated with the delimitable partial amounts;
- admixing the amplification product with catcher molecules on a DNA chip, so that hybrids of catcher molecules and target sequences are formed from the amplification product, wherein the DNA chip includes at least two groups of spots, wherein the spots within a group have different catcher molecules and each group of spots is associated with one of the delimitable partial amounts of the genetic material;
- quantitatively detecting the hybrids formed in each case in a spot with different catcher molecules of the DNA chip so that a respective detection value is obtained for each spot;
- averaging the detection values of the groups of spots present on the DNA chip;
- determining the relative frequency of partial amounts of genetic material within a genetic material by comparing the averages.

10. A method in accordance with any one of the claims 1 - 9, **characterized in that** the genetic material stems from or is traceable back to a single cell.

11. A method in accordance with any one of the claims 1 - 9, **characterized in that** the genetic material is a chromosome set from a polar body of an egg cell.

12. A method in accordance with any one of the claims 1 - 9, **characterized in that** a delimitable partial amount consists of one or more chromosomes.

13. A method in accordance with any one of the claims 1 - 9, **characterized in that** a delimitable partial amount consists of one or more genes.

14. A method in accordance with any one of the claims 1 - 9, **characterized in that** genetic information of a genetic reference material is amplified in parallel, under otherwise identical reaction conditions, so that an amplification product is obtained which substantially only has amplified sequences which include specific target sequences of predetermined length and specific to the respective genetic information, which is suitable for detection by means of hybridization.

15. A method in accordance with any one of the claims 1 - 14, in which the primer for the amplification step is primer Ale1-k having the base sequence 5'-CCAAAGTGCTGGGATTACAG-3'.

## Revendications

1. Méthode de détermination de la fréquence relative de portions délimitables d'un matériel génétique, avec amplification du matériel génétique par réaction en chaîne de la polymérase, méthode dans laquelle sont utilisées des amorces complémentaires de sites de liaison d'amorces qui sont présents en plusieurs points dans le matériel génétique et dont chacun est contigu à une séquence cible de longueur prédéterminée à chaque fois spécifique d'une portion, ce qui permet d'obtenir un produit d'amplification qui ne présente principalement que des séquences amplifiées qui comprennent une séquence cible de longueur prédéterminée, spécifique de l'information génétique respective, qui convient pour la détection par hybridation, le produit d'amplification étant analysé au moyen d'un essai d'hybridation visant à déterminer la fréquence relative des portions délimitables.

2. Méthode suivant la revendication 1,
**caractérisée en ce que** l'homologie entre les amorces et les sites respectifs de liaison des amorces se situe dans une plage de 80 % à 100 %, et avantageusement dans une plage de 90 % à 100 %.

3. Méthode suivant l'une des revendications 1-2,
**caractérisée en ce que** des éléments structuraux nucléotidiques pourvus de marquages sont utilisés au cours de la réaction en chaîne de la polymérase.

4. Méthode suivant l'une des revendications 1-3,
**caractérisée par**
- un mélange du produit d'amplification avec des molécules pièges, de manière qu'il se forme des hybrides entre les molécules pièges et les séquences cibles spécifiques, et
- la détection des hybrides.

5. Méthode suivant la revendication 4,
**caractérisée en ce que** les molécules pièges sont disposées sur une puce d'ADN.

6. Méthode suivant la revendication 4,
**caractérisée en ce que** les molécules pièges sont formées d'oligonucléotides.

7. Méthode suivant l'une des revendications 1-6,
**caractérisée en ce qu'**il y est utilisé une puce d'ADN pour laquelle de mêmes molécules pièges sont à chaque fois prévues dans un spot individuel.

8. Méthode suivant l'une des revendications 1-7,
**caractérisée en ce qu'**il y est utilisé une puce d'ADN pour laquelle sont prévues, dans un spot individuel, différentes molécules pièges spécifiques pour différentes séquences cibles qui sont toutes coordonnées à l'une des portions délimitables du matériel génétique.

9. Méthode de détermination de la fréquence relative de portions délimitables d'un matériel génétique suivant l'une des revendications 1-8, comprenant les étapes suivantes :
- mise en oeuvre d'un processus d'amplification d'informations génétiques à partir d'un matériel génétique qui comprend plusieurs portions délimitables les unes par rapport aux autres de manière à obtenir un produit d'amplification comportant des séquences cibles qui conviennent pour une détection par hybridation et qui peuvent être coordonnées aux portions délimitables,
- mélange du produit d'amplification avec des molécules pièges portées par une puce d'ADN de manière à former des hybrides des molécules pièges et de séquences cibles à partir du produit d'amplification, la puce d'ADN comprenant au moins deux groupes de spots dont les spots à l'intérieur d'un groupe présentent des molécules pièges différentes, et un groupe de spots est coordonné dans chaque cas à l'une des portions délimitables du matériel génétique,
- détection quantitative des hybrides formés à chaque fois dans un spot avec différentes molécules pièges de la puce d'ADN, de manière à obtenir dans chaque cas une valeur de détection pour chaque spot,
- formation de la moyenne des valeurs de détection des groupes de spots présents sur la puce d'ADN,
- détermination de la fréquence relative de portions de matériel génétique à l'intérieur d'un matériel génétique par comparaison des valeurs moyennes.

10. Méthode suivant l'une des revendications 1-9,
**caractérisée en ce que** le matériel génétique provient d'une cellule individuelle ou doit y être rapporté.

11. Méthode suivant l'une des revendications 1-9,
**caractérisée en ce que** le matériel génétique est un ensemble de chromosomes provenant d'un corpuscule polaire d'un ovule.

12. Méthode suivant l'une des revendications 1-9,
**caractérisée en ce qu'**une portion délimitable est constituée d'un ou plusieurs chromosomes.

13. Méthode suivant l'une des revendications 1-9,
**caractérisée en ce qu'**une portion délimitable est constituée d'un ou plusieurs gènes.

14. Méthode suivant l'une des revendications 1-9,
**caractérisée en ce que** des informations génétiques d'un matériel génétique de référence sont amplifiées en parallèle dans des conditions réactionnelles autrement identiques de manière à obtenir un produit d'amplification qui ne présente principalement que des séquences amplifiées comprenant des séquences cibles de longueur prédéterminée spécifiques de l'information génétique respective, qui conviennent pour une détection par hybridation.

15. Méthode suivant l'une des revendications 1-14, dans laquelle l'amorce Ale1-k présentant la séquence de bases 5'-CCAAAGTGCTGGGATTACAG-3' est utilisée pour l'amplification.
